# EUROPEAN PATENT SPECIFICATION

(11) **EP 3 344 299 B1**
(45) Date of publication and mention of the grant of the patent: **11.08.2021**
(21) Application number: 16767159.3
(22) Date of filing: 01.09.2016
(51) Int. Cl.: A61L 24/00, A61L 24/10

(54) **HEMOSTATIC MATERIAL**
HÄMOSTATISCHES MATERIAL
MATÉRIAU HÉMOSTATIQUE

(30) Priority: 01.09.2015 EP 15183295
(43) Date of publication of application: 11.07.2018
(73) Proprietor: Baxter International Inc, Deerfield, IL 60015 (US)
(72) Inventor: QIN, Xiao-Hua, 1200 Vienna (AT); SLEZAK, Paul, 1050 Vienna (AT); REDL, Heinz, 1060 Vienna (AT)
(74) Representative: Sonn & Partner Patentanwälte
(86) International application number: PCT/EP2016/070619
(87) International publication number: WO 2017/037178

(56) References cited:
- WO-A1-96/40033
- WO-A2-03/057072

### SEQUENCE LISTING

<110> TRAUMA CARE CONSULT Traumatologische Forschung Gemeinnützige Gesellschaft mbH
<120> Hemostatic Material
<130> R 69873
<150> EP 15183295.3
   <151> 2015-09-01
<160> 15
<170> PatentIn version 3.5
<210> 1
   <211> 6
   <212> PRT
   <213> Artificial Sequence
<220>
   <223> synthetic peptide derived from natural protein
<400> 1
   Ser Phe Leu Leu Arg Asn
   1               5
<210> 2
   <211> 23
   <212> PRT
   <213> Artificial Sequence
<220>
   <223> synthetic peptide derived from natural protein
<400> 2
   Ala Gly Tyr Lys Pro Asp Glu Gly Lys Arg Gly Asp Ala Cys Glu Gly
   1               5                   10                  15
   Asp Ser Gly Gly Pro Phe Val
               20
<210> 3
   <211> 14
   <212> PRT
   <213> Artificial Sequence
<220>
   <223> synthetic peptide derived from natural protein
<400> 3
   Arg Gly Asp Ala Cys Glu Gly Asp Ser Gly Gly Pro Phe Val
   1               5                   10
<210> 4
   <211> 14
   <212> PRT
   <213> Artificial Sequence
<220>
   <223> synthetic peptide derived from natural protein
<400> 4
   Ser Phe Leu Leu Arg Asn Pro Asn Asp Lys Tyr Glu Pro Phe
   1               5                   10
<210> 5
   <211> 13
   <212> PRT
   <213> Artificial Sequence
<220>
   <223> synthetic peptide derived from natural protein
<400> 5
   Ser Phe Leu Leu Arg Asn Pro Asn Asp Lys Tyr Glu Pro
   1               5                   10
<210> 6
   <211> 12
   <212> PRT
   <213> Artificial Sequence
<220>
   <223> synthetic peptide derived from natural protein
<400> 6
   Ser Phe Leu Leu Arg Asn Pro Asn Asp Lys Tyr Glu
   1               5                   10
<210> 7
   <211> 11
   <212> PRT
   <213> Artificial Sequence
<220>
   <223> synthetic peptide derived from natural protein
<400> 7
   Ser Phe Leu Leu Arg Asn Pro Asn Asp Lys Tyr
   1               5                   10
<210> 8
   <211> 10
   <212> PRT
   <213> Artificial Sequence
<220>
   <223> synthetic peptide derived from natural protein
<400> 8
   Ser Phe Leu Leu Arg Asn Pro Asn Asp Lys
   1               5                   10
<210> 9
   <211> 9
   <212> PRT
   <213> Artificial Sequence
<220>
   <223> synthetic peptide derived from natural protein
<400> 9
   Ser Phe Leu Leu Arg Asn Pro Asn Asp
   1 5
<210> 10
   <211> 8
   <212> PRT
   <213> Artificial Sequence
<220>
   <223> synthetic peptide derived from natural protein
<400> 10
   Ser Phe Leu Leu Arg Asn Pro Asn
   1 5
<210> 11
   <211> 7
   <212> PRT
   <213> Artificial Sequence
<220>
   <223> synthetic peptide derived from natural protein
<400> 11
   Ser Phe Leu Leu Arg Asn Pro
   1 5
<210> 12
   <211> 6
   <212> PRT
   <213> Artificial Sequence
<220>
   <223> synthetic peptide derived from natural protein
<400> 12
   Ser Leu Ile Gly Lys Val
   1 5
<210> 13
   <211> 6
   <212> PRT
   <213> Artificial Sequence
<220>
   <223> synthetic peptide derived from natural protein
<400> 13
   Thr Phe Arg Gly Ala Pro
   1 5
<210> 14
   <211> 6
   <212> PRT
   <213> Artificial Sequence
<220>
   <223> synthetic peptide derived from natural protein
<400> 14
   Gly Tyr Pro Gly Gln Val
   1 5
<210> 15
   <211> 9
   <212> PRT
   <213> Artificial Sequence
<220>
   <223> synthetic peptide derived from natural protein
<400> 15
   Ser Phe Leu Leu Arg Asn Pro Asn Cys
   1 5

## Description

The present invention relates to hemostatic material and methods for producing and using such materials.

Uncontrolled bleeding is still the leading cause of mortality in traumatic and surgical injuries.^{[1]} Developing effective therapeutic approaches to control bleeding is therefore of paramount clinical and social values. In the last decades, a number of hemostatic products^{[2]} have been developed, including fibrin-based glue or sealants, ^{[3 a,b]} zeolite powders, ^{[4 a,b]} crosslinked gelatin matrix^{[5 a,b]} and so forth. However, each of these products has its respective limitations. Fibrin products suffer from high cost, short shelf-life and weak mechanical strength.^{[6]} Zeolite minerals are prone to cause severe burns and are not degradable.^{[6]} Crosslinked gelatin matrix could halt bleeding within minutes only when combined with high doses of thrombin. ^{[7]} However thrombin is unstable in solution due to autoproteolysis. Highly concentrated thrombin is known to induce apoptosis of human keratinocytes and can cause impairments in wound healing.^{[8]} Hence, there exists a strong need to design alternative hemostatic materials with improved safety. In particular, designing an effective strategy that avoids the use of highly concentrated thrombin is a desirable solution.

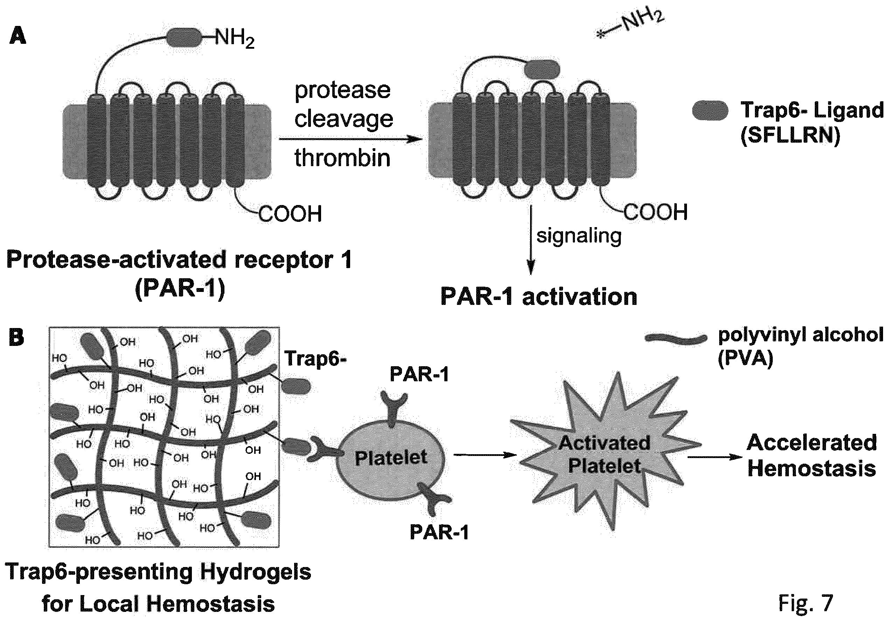

Thrombin is a serine protease that plays important roles in blood clotting (coagulation). ^{[9 a,b]} As the key coagulation protease, thrombin converts soluble fibrinogen into fibrin networks crosslinked by a transglutaminase (FXIII).^{[10]} In addition, thrombin is the most potent activator of platelets by stimulating protease-activated receptors (PAR).^{[11, 12]} Upon activation by thrombin, platelets physically alter the conformation of GP IIb/IIIa receptors and provide high-affinity binding sites for fibrinogen, providing fibrinogen-crosslinked platelet aggregation. Both PAR-1 and PAR-4 are present on human platelets, yet activation of human platelets by thrombin is primarily mediated by PAR-1.^{[13]} The molecular mechanism of PAR-1 activation by thrombin is depicted in Fig. 7A. PAR-1 is highly expressed in platelets, ^{[14 a, b]} and PAR-1 activation is initiated by proteolytic cleavage of part of the extracellular N-terminal domain of PAR-1 receptor by thrombin. Proteolysis generates new N-terminal ligand domains (SFLLRN (SEQ ID NO:1), a.k.a. thrombin receptor agonist peptide-6, TRAP6) that interact with the receptor within the extracellular loop 2 and triggers the signaling pathway of PAR-1 activation. It has been proven that short TRAP6 peptide (SFLLRN) could work as a potent platelet activator separately and stimulates platelet aggregation via PAR-1 signaling.^{[15]} Multiplate® TRAP test has become a standard in vitro assay in whole blood or in platelet rich plasma for quantitative determination of platelet function triggered by TRAP6. TRAP test allows analysis of platelet function activated through PAR-1 signaling without triggering fibrin formation, which otherwise occurs when thrombin is the agonist, because of using a thrombin inhibitor in the sample.

WO 96/40033 A1 discloses a hemostatic material with hemostatic agents, including epsilon aminocaproic acid and a thrombin receptor activating peptide, wherein the hemostatic agents are sprayed or coated on a hemostatic matrix so as to obtain a matrix wherein the agent is physically (but not covalently) adsorbed on the matrix. Such patches have the drawback that the hemostatic agents provided therewith easily release from the patch when contacted to a bleeding area of a patient. Such there is the potential danger of inducing systemic thrombotic events, especially since there are no circulating antagonists in the circulation.

WO 03/057072 A2 discloses hemostatic compositions comprising cellulose and a polysaccharide covalently linked thereto.

It is an object of the present invention to provide improved hemostatic material with thrombin receptor activating agents for controlling bleeding.

Therefore, the present invention provides a hemostatic material, wherein a thrombin receptor activating agent is covalently coupled to a biocompatible matrix and wherein the thrombin receptor activating agent has a thrombin receptor binding activity after covalent coupling to the biocompatible matrix.

With the present invention it is shown for the first time that a thrombin receptor activating agent can be covalently immobilized on a biocompatible matrix so as to obtain an improved hemostatic material suitable for administration to human patients in need thereof. As a preferred embodiment, covalent coupling of TRAP6, TRAP7 or TRAP8 to a synthetic hydrogel matrix (e.g. a polyvinyl alcohol based polymer) resulted in a suitable hemostatic material, maintaining the activity for platelet activation in a safe, localized manner over a considerable period of time so as to enable an improved hemostasis, especially via an induced platelet aggregation.

The biocompatible matrix according to the present invention may be any matrix that is useable for being administered to human patients, especially for wound coverage or filling of volumetric defects (e.g. in organs) of a human patient. According to the present invention, it is preferred to use the matrix materials that have been suggested in the prior art for such purposes. In general, a "biocompatible" matrix is a matrix that may be administered to human patients and that does not induce a negative effect in the course of this administration and contact with the patient. A "biocompatible" matrix is a matrix that does not contain materials or components that threaten, poison, impede, or adversely affect living tissue (e.g. human tissue that is exposed to the surface in wounds). Examples for such matrices are "classical" wound coverages, such as patches, sponges, but also flowable or sprayable matrices, powders, etc. such as FloSeal™ (a cross-linked gelatin matrix), Surgiflo™ (a bovine gelatin paste). Whereas patches are advantageous for general wound coverages, non-material hemostats, most prominently flowable matrices, can be delivered within the same phase as opposed to liquid/solid approaches or can be used to flexibly fill cavities or provide a flexible scaffold ("volumetric defects"). The matrix should be chemically active or chemically activated so that the thrombin receptor activating agent can be covalently coupled to the matrix according to the present invention. For example, the matrix may have hydroxyl groups, vinyl groups, carboxyl groups, or amino groups to allow covalent attachment of the thrombin receptor activating agent to the matrix.

Preferably, the matrix is a hemostatic matrix, i.e. the matrix material as such has already hemostatic properties. Such materials are well available in the art and comprise e.g. collagen, gelatin or chitosan.

A preferred biocompatible matrix is selected from the group consisting of a biomaterial, preferably a protein, a biopolymer or a polysaccharide matrix, especially a collagen, gelatin, fibrin, starch or chitosan matrix; and a synthetic polymer, preferably a polyvinyl alcohol, polyethylene glycol, poly(N-isopropylacrylamide), etc..

Preferably, the matrix of the present invention is biodegradable, i.e. it is naturally absorbed by the patient's body after some time. In any way, the material (including the matrix) must be biocompatible, i.e. have no harming effect to the patient to whom the material is administered. Such biodegradable materials are specifically suitable in situations where hemostasis is achieved inside the body, i.e. in the course of surgery and the site is closed after surgery.

Accordingly, the matrix is preferably a biomaterial selected from biopolymers such as a protein, or a polysaccharide. Especially preferred is a biomaterial selected from the group consisting of collagen, gelatin, fibrin, a polysaccharide, e.g. hyaluronic acids, chitosan, and a derivative thereof, more preferred collagen and chitosan, especially preferred collagen. Such collagen matrix used for the present invention can be derived from any collagen suitable to form a gel, including a material from liquid, pasty, fibrous or powdery collagenous materials that can be processed to a porous or fibrous matrix as well as particles. The preparation of a collagen gel for the production of a sponge or sheet may include acidification until gel formation occurs and subsequent pH neutralisation. To improve gel forming capabilities or solubility the collagen may be (partially) hydrolyzed or modified, as long as the property to form a stable sponge or sheet when dried is not diminished. The matrix used for coupling the thrombin receptor activating agent can be a biopolymer, i.e., a naturally occurring polymer or a derivative thereof, or can be a synthetic polymer. Examples of biopolymers useful in a hemostatic material according to the present invention include polypeptides such as collagen, collagen derivatives such as gelatin, elastin, and elastin derivatives, and polysaccharides such as hyaluronic acids, starch, cellulose, or a derivative thereof, for example, oxidized cellulose. Preferably, the biopolymer is a human biopolymer, which can be isolated from an individual or can be a synthetic biopolymer, e.g., a recombinantly produced biopolymer.

In various embodiments, the matrix comprises a recombinant human polymer. In particular, the recombinant human polymer can be a recombinant human collagen, such as, for example, recombinant human collagen type I, recombinant human collagen type III, or a combination thereof. In one embodiment, the matrix comprises recombinant human collagen type III. In another embodiment, the matrix comprises recombinant human collagen type I. For example, the recombinant human gelatin can be derived from recombinant human collagen type III. In yet another embodiment, the matrix comprises recombinant gelatin derived from recombinant human collagen type I. In further embodiments, the matrix comprises recombinant gelatin produced directly by expression of encoding polynucleotide

The polysaccharide used as a matrix in the present invention is preferably selected from the group consisting of cellulose, alkyl cellulose, methylcellulose, alkylhydroxyalkyl cellulose, hydroxyalkyl cellulose, cellulose sulfate, salts of carboxymethyl cellulose, carboxymethyl cellulose, carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, chitosan, heparin, heparin sulfate, heparan, heparan sulfate, dermatan sulfate, keratan sulfate, carrageenans, chitosan, starch, amylose, amylopectin, poly-N-glucosamine, polymannuronic acid, polyglucuronic acid, polyguluronic acid, derivatives of said polysaccharides, or combinations thereof.

The present matrix may also be based on a synthetic polymer. The synthetic absorbable polymer can be an aliphatic polyester polymer, an aliphatic polyester copolymer, or combinations thereof.

The present matrix may also be provided in the form of a woven or non-woven fabric made of fibers. Such fibers are preferably made of a biocompatible and/or biodegradable material. A number of such fibers have been used so far to provide hemostatic fabrics. In some embodiments, such nonwoven or woven fibers may comprise one or more polysaccharides such as pectin, acetylated pectin, hyaluronic acid and derivatives of thereof, and the like. In some embodiments, the pectin and/or acetylated pectin may be derived from sugar beets. In other embodiments, the polysaccharide may be a non-cellulosic polysaccharide. The woven or nonwoven fibers may also include fibers comprising other biodegradable polymers including polyglycolide, polylactide, poly(lactide-co-glycolide), poly(t-caprolactone), poly(dioxanone), polycaprolactone, poly(3-hydroxybutyric acid), poly(3- hydroxybutyric acid-co-3 -hydroxy valeric acid), alginates, collagen, chitosan, gelatin, fibrinogen, elastin, polyethers, polyanhydrides, polyesters, polyorthoesters, polyphosphazenes, polyvinyl alcohol, polyvinylpyrrolidone, polytrimethylene carbonate, and the like. In addition, natural protein fibers such as cotton, silk and wool may also be used.

The matrix material according to the present invention may preferably be provided as granules of various morphologies, including powder or matrices for flowable hemostats. For example, the granules may have a (median particle) size of 1 to 1.000 µm, preferably from 10 to 1.000 µm, especially from 200 to 800 µm. Suitable matrices as flowable hemostats are disclosed e.g. in WO 98/08550 A or WO 2003/007845 A.

According to a specifically preferred embodiment, the present invention uses polyvinyl alcohol (PVA) as a matrix material. PVA is a water-soluble polymer originated from partial hydrolysis of polyvinyl acetate. PVA-based hydrogels used as a matrix according to the present invention have been widely used in tissue engineering and drug delivery systems because of their superior biocompatibility (FDA-approved). ^{[16 a,b]} In addition, PVA hydrogels are well known for being uniquely stronger than most other synthetic hydrogels.

The present invention uses thrombin receptor activating agents covalently bound to a hemostatically suitable matrix. Preferred embodiments of thrombin receptor activating agents are thrombin receptor activating peptides (TRAPs). TRAPs are a family of peptides of varying amino acid length which correspond to the new N-terminal region of the thrombin receptor. These synthetic or recombinant peptides mimic the activated form of the extracellular portion of the thrombin receptor protein and function as thrombin agonists.

US 5,256,766 A and WO 96/40033 A1 describe pharmaceutical compounds and hemostatic patches containing TRAPs or "agonists" as useful to encourage blood clotting, for example, in localized application at internal bleeding sites of hemophiliacs. The agonists are disclosed as mimicking thrombin's ability to stimulate fibroblast proliferation and, concomitantly, platelet aggregation. TRAPs thus can be useful in promoting hemostasis and wound healing. With the use of TRAPs, an effective hemostatic material and hemostatic bandage can be provided which can be completely free of biological compounds such as thrombin and fibrinogen and the concomitant dangers of viral contamination.

Representative TRAPs which may be incorporated into a material according to the present invention include peptides capable of activating thrombin receptor, such as the agonists identified in the US 5,256,766 A by the formula AAx --AAy-(AAi)n--z.

Other TRAPs which have been disclosed which activate fibroblasts and are implicated in wound healing include peptides TRAP 508-530, amino acids AGYKPDEGKRGDACEGDSGGPFV (SEQ ID NO:2); and TRAP 517-530, amino acids RGDACEGDSGGPFV (SEQ ID NO:3). Further suitable TRAPs are disclosed by Carney et al. J. Clin Invest. 89:14691477 (1992); Furman et al. PNAS 95 (1998), 3082-3087 and Cromack et al., J. Surg. Res.53: 117 (1992). Accordingly, suitable TRAPs useful in the present invention, for example, include peptides SFLLRNPNDKYEPF (SEQ ID NO:4), SFLLRNPNDKYEP (SEQ ID NO:5), SFLLRNPNDKYE (SEQ ID NO:6), SFLLRNPNDKY (SEQ ID NO:7), SFLLRNPNDK (SEQ ID NO:8), SFLLRNPND (SEQ ID NO:9), SFLLRNPN (TRAP8 (SEQ ID NO:10)), SFLLRNP (TRAP7 (SEQ ID NO:11)), SFLLRN (TRAP6), SFLLR, SFLL, and SFL, and the amidated forms thereof. Because TRAPs are small peptides, they are more stable than large proteinaceous platelet activating agents, such as thrombin. The stability of TRAPs contributes to the properties of the present material which permit it to be stored without refrigeration.

Preferably, the thrombin receptor activating agents (preferably the TRAP) is provided with a linker so as to covalently couple the TRAP to the matrix. Preferred linkers are amino acids (single amino acids, such as Cysteine, Arginine, Lysine, Serine, Glycine, etc. (preferably Cysteine), or short amino acid linkers with e.g. 2 to 5 amino acid residues, preferably comprising amino acids selected from the group of Cysteine, Arginine, Lysine, Proline, Asparagine, Glutamine, Serine and Glycine. Preferred dipeptidic linkers may be Cys-Gly-(the terminal "-" indicates the bond to the thrombin receptor activating agent), -Gly-Cys, Cys-Arg-, -Arg-Cys, -Asn-Cys, Cys-Asn-, -Pro-Cys, Cys-Pro-; preferred tripeptidic linkers may be Cys-Gly-Gly-, -Gly-Gly-Cys, -Pro-Asn-Cys, Cys-Asn-Pro-, Cys-Pro-Asn-, -Asn-Pro-Cys etc., or any other peptidic linker comprising 2 to 5 amino acid residues known for pharmaceutical peptide coupling to carriers or matrices.

According to a specifically preferred embodiment, the thrombin receptor activating agent of the present material is a thrombin receptor activating peptide (TRAP), preferably TRAP8, TRAP7, TRAP6, TRAP1-41, SLIGKV (for PAR-2 (human) (SEQ ID NO:12)), TFRGAP (for PAR-3 (human) (SEQ ID NO:13)), GYPGQV (for PAR-4 (human) (SEQ ID NO:14)), or amidated forms thereof, as well as mixtures of such agents.

The hemostatic material according to the present invention may have any suitable form that is usable for the treatment of human patients in need of a hemostatic material, i.e. as a flowable or sprayable form; as a two-dimensional form (where the third dimension extension is comparably small (e.g. less than 1/10 or 1/20) compared to the other two dimensions; or as a three-dimensional form (e.g. a sponge, a paste, a cavity implant, etc.). A preferred two- or three-dimensional embodiment of the material according to the present invention may, for example, be a sponge, a woven or non-woven fabric, a preformed shape, preferably as a cylinder or cone (e.g. for tooth extraction) or as being used as a flexible or non-flexible scaffold, a particulate or granulate material or a sheet. It is specifically preferred if the matrix is able to absorb fluid from the wound so as to attract further blood coagulation components from the wound once the material is applied to the wound to achieve platelet aggregation. Furthermore, the material is preferably flexible and suitable to be applied on diverse tissues and locations with various shapes.

Further preferred embodiments of the hemostatic material according to the present invention comprise further ingredients, such as anti-bacterial agents, coagulatively active agents, immunosuppressive agents, anti-inflammatory agents, anti-fibrinolytic agents, such as aprotinin or ECEA, growth factors, vitamins, cells, etc.. In a preferred embodiment, however, the material according to the present invention may or may not have such further ingredients, provided that the material is free of components which could have negative impact on the storability or administration of the hemostatic material. Accordingly, the present hemostatic material is preferably essentially free of any protein degrading activity, especially free of protease activity, specifically free of thrombin activity. Thrombin is added frequently to hemostatic materials in order to promote fibrin cleavage and clot formation; however, it may also be proteolytic to the hemostatic material, which may be unwanted especially during production and storage of such material. With the present invention, the addition or presence of thrombin or comparable components is not required and may therefore be omitted without negative impact on the hemostatic properties of the hemostatic material.

Preferably, the hemostatic material according to the present invention is provided in a state wherein it is able to soak up liquid material, such as blood. The ability to soak up blood (and the components therein promoting clot formation, bleeding termination and wound closure) significantly enhances the overall efficacy of the hemostatic material. For example, the hemostatic material according to the present invention is provided in a dry form or in a wet form still allowing the material to take up further liquid material (i.e. being soaked with liquid in an amount which amount is still under its soaking capacity). This allows blood entering and/or passing through the hemostatic material so as to provide the blood components useful e.g. in the wound closure process in an enlarged volume or in the whole (or virtually the whole) volume of the material applied.

According to a further aspect, the present invention relates to a method for producing the hemostatic material according to the present invention. This method for producing a hemostatic material is characterised by the step of covalently coupling a thrombin receptor activating agent to a hemostatic matrix; wherein the thrombin receptor activating agent is activated with peptide sequences with bioorthogonal groups, and/or wherein the biocompatible matrix is activated with an ene group.

Preferably, the thrombin receptor activating agent is a thrombin receptor activating peptide (TRAP), preferably TRAP8, TRAP7, TRAP6, TRAP1-41, SLIGKV (for PAR-2 (human)), TFRGAP (for PAR-3 (human)), GYPGQV (for PAR-4 (human)), or amidated forms thereof, as well as mixtures of these agents.

It is, of course, important for the present invention that the thrombin receptor activating agent keeps its thrombin receptor activating activity after covalent coupling to the biocompatible matrix. Not any biomolecule can be simply bound to a biocompatible surface and still retain its bio-functionality. In the course of generating the present invention, it turned out that the usage of conventional binding techniques to covalently couple C- or N-terminus of the thrombin receptor activating agents according to the present invention (e.g. TRAP6 peptides) usually results in the loss of said bioactivity. Accordingly, the provision of covalent immobilization approach that indeed retains full bio-functionality of the thrombin receptor activating agent (which is a peptide) is not trivial and needs to rely on the disclosure to obtain such functional embodiments contained herein.

For example, use of traditional coupling techniques for peptides, such as those using EDC (1-Ethyl-3-(3-dimethylaminopropyl)carbodiimide; reaction of C-terminus, Asp and Glu) or NHS (N-hydroxysuccinimide; reaction of N-terminus, Lys), did not result in active immobilised thrombin receptor activating agent (because they are usually non-specific, prone to side reaction with acidic amino acids or lysine, deactivation of peptides that relies on N-terminus, etc. ^{[29], [30], [31]}). Techniques using cysteine and/or Michael addition also carry the risk of loss of function due to possible side reactions with amine groups^{[32]}. Accordingly, such traditional coupling techniques result in loss of activity. While a variety of chemical approaches for peptide immobilization have been reported^{[34]}, most of these methods rely on reactions with carboxyl groups at the C-terminus (e.g. EDC) ^{[29]}, or with primary amines at the N-terminus (e.g. NHS) ^{[30], [31]}, or with cysteine residues via Michael-addition based on maleimide or vinyl sulfone groups ^{[32]}. Unfortunately, most reported reactions are non-specific and prone to side reactions, either with acidic/basic amino acids (for EDC/NHS) or with amine groups (for Michael addition), which induce unfavorable loss of peptide activity. For instance, N-hydroxysuccinimide (NHS) ester conjugation is the most often used approach to covalently immobilize bioactive peptides (e.g. cell-adhesive RGD) onto polymer substrates through reacting with the N-terminus of peptide ^{[35]}. Although primary amines are the most reactive groups for NHS esters, recent studies have demonstrated that a series of side reactions could occur with other peptide residues (e.g. -OH for tyrosine, serine, threonine, guanidinium for arginine) ^{[30],[31]}. Furthermore, previous studies on the structure-function relationship of TRAPs (SFLLRN-), highlighted the significance of the amino acids at the N-terminus to maintain peptide activity^{[36]}. With these considerations in mind, it has been found that it is important for the present invention to develop a peptide immobilization approach that circumvents side reactions with the N-terminus of the thrombin receptor activating agent (being a peptide, such as TRAP6), in order to retain its bioactivity.

The present invention therefore provides a thrombin receptor activating active agent in immobilised form with retained activity. This turned out to be not feasible to be provided by conventional state-or-the-art approaches for peptide immobilization. The present invention therefore also provides a selection of specific coupling techniques (referred to above) with no risk of side reactions with N- or C-terminus or acidic or basic amino acids by using e.g. bioorthogonal reactions, such as photo-click conjugation of cysteine-containing TRAP6 onto PVA norbornenes, which offers a very high degree of conjugation efficiency (>95%), site-specificity and modularity. It is clear that the same conjugation approach is also applicable for other substrates bearing norbornene groups, such as naturally-derived molecules (gelatin, hyaluronan, alginate, etc.) and synthetic analogues such as PEG. In addition, the polymer-bound thrombin receptor activating active peptide conjugates have lower probability to be internalized by blood cells than soluble forms thereof through PAR-1 receptor signalling, as the size of polymer substrates applied as biocompatible solid matrix for the present invention, such as PVA substrates (hundreds of repeating units), is far larger than a short TRAP6 sequence. In all, the approach according to the present invention with coupling the thrombin receptor activating active agent with retained activity to a biocompatible solid matrix (e.g. by photo-click conjugation for TRAP6 peptide immobilization) provides significant practical values for local hemostasis as well as other medical applications.

According to a preferred embodiment of this method, the hemostatic matrix is functionalized with chemical groups (e.g., alkene, sulfhydryl, alkyne, azido, hydroazide, hydrazine), preferably groups that allow high-efficiency covalent binding of the thrombin receptor activating agent via bioorthogonal reactions (e.g., thiol-ene addition, alkyne-azide cycloaddition, Diels-Alder reaction, hydrazide-hydrazine reaction).

Preferably, the thrombin receptor activating agent is modified with peptide sequences that are engineered with bioorthogonal groups (e.g., alkene, sulfhydryl, alkyne, azido, hydroazide, hydrazine), especially with a cysteine moiety with an -SH group.

In a preferred embodiment of the method according to the present invention, the hemostatic material is functionalized with an ene group, such as norbornene, maleimide, allyl, vinyl ester, acrylate, vinyl carbonate, methacrylate, etc.

According to a specifically advantageous embodiment of the present method, the chemical coupling is performed by photo-induced reactions, especially by radical-mediated thiol-(norborn-)ene photo-click chemistry (reviewed in [18^{a}]) or (other) photo-triggered click chemistry (reviewed in [18^{b}]).

The present hemostatic material may be finished as a commercial product by the usual steps performed in the present field, for example by appropriate sterilisation and packaging steps. For example, the present material may be treated by UV/vis irradiation (200-500 nm), preferably with the help of photoinitiators with different absorption wavelengths (e.g. Irgacure 184, 2959), preferably water-soluble initiators (Irgacure 2959). Such irradiation is usually performed for an irradiation time of 1-60 min, but also longer irradiation times may be applied, depending on the specific method. The material according to the present invention may be finally sterile-wrapped so as to retain sterility until use and packaged (e.g. by the addition of specific product information leaflets) into suitable containers (boxes, etc.).

According to another aspect, the present invention also relates to the hemostatic material according to the present invention for use in surgery and/or in the treatment of injuries and/or wounds. The hemostatic material according to the present invention is specifically suitable and effective for increasing the release of platelet-derived growth factors and for accelerating wound healing.

This makes the hemostatic material according to the present invention an excellent tool for sealing of anastomosis, for suture line sealing and to safeguard hemostasis in resection sites.

According to another aspect of the present invention, the hemostatic material according to the present invention may also be provided in kit form combined with other components necessary for administration of the material to the patient. For example, if the hemostatic material may be provided in flowable dry form (e.g. as granules or as a powder) or as a flowable paste, it is preferred to provide such material with a suitable buffer solution which can be added shortly before administration to the patient. Such a buffer solution usually contains (besides the buffer components, such as phosphate, carbonate, TRIS, etc. buffer systems) divalent metal ions, preferably Ca²⁺ ions, or other functional components (if not already present on or in the matrix), such as anti-bacterial agents, coagulatively active agents, immunosuppressive agents, anti-inflammatory agents, anti-fibrinolytic agents, such as aprotinin or ECEA, growth factors, vitamins, cells, etc.. The kit may further contain means for administering or preparing administering the hemostatic material, such as syringes, tubes, catheters, forceps, scissors, sterilising pads or lotions, etc..

Accordingly, the present invention relates to a kit, preferably for use in surgery and/or in the treatment of injuries and/or wounds, comprising
- a hemostatic material according to the present invention and
- at least one administration device, preferably selected from the group buffer solution, especially a buffer solution containing Ca²⁺ ions, a syringe, a tube, a catheter, forceps, scissors, a sterilising pad or lotion.

Preferably, the buffer solution further comprises a component selected from the group anti-bacterial agent, coagulatively active agent, immunosuppressive agent, anti-inflammatory agent, anti-fibrinolytic agent, especially aprotinin or ECEA, growth factor, vitamin, cell, or mixtures thereof. Alternatively, the kit may also further comprise a container with a component selected from the group anti-bacterial agent, coagulatively active agent, immunosuppressive agent, anti-inflammatory agent, anti-fibrinolytic agent, especially aprotinin or ECEA, growth factor, vitamin, cell, or mixtures thereof.

The present invention is further illustrated by the following examples and the figures, yet without being restricted thereto.

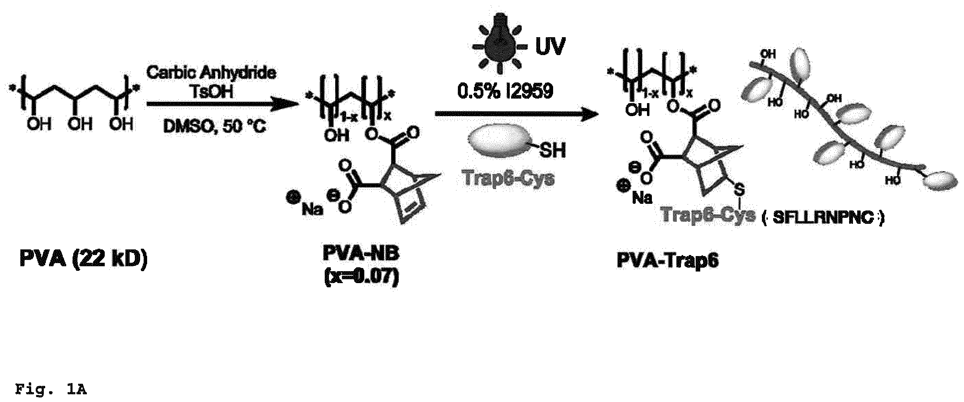

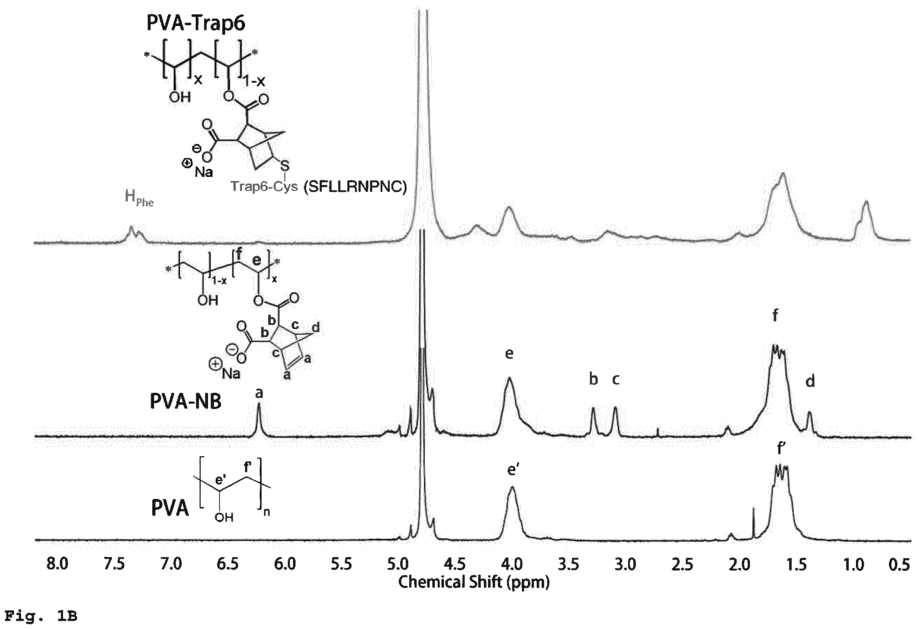

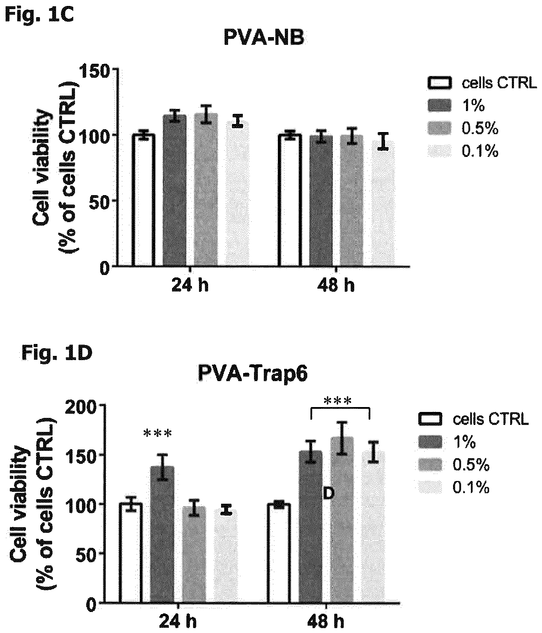

Fig. 1 shows (A) synthesis scheme of PVA-NB and PVA-TRAP6 (reaction in abs. DMSO at 50 °C for 12 h, TsOH: p-Toluenesulfonic acid, 12959: Irgacure 2959, i.e. one commonly used water-soluble PI); (B) ¹H-NMR (D₂O) spectra of PVA, PVA-NB and PVA-TRAP6 conjugate; and normalized viability of C2C12 cells after 24/48 h exposure to PVA-NB, (C) and PVA-TRAP6 solutions (D) with varying polymer concentrations (1%, 0.5%, and 0.1%) investigated by MTT assay (n>3).

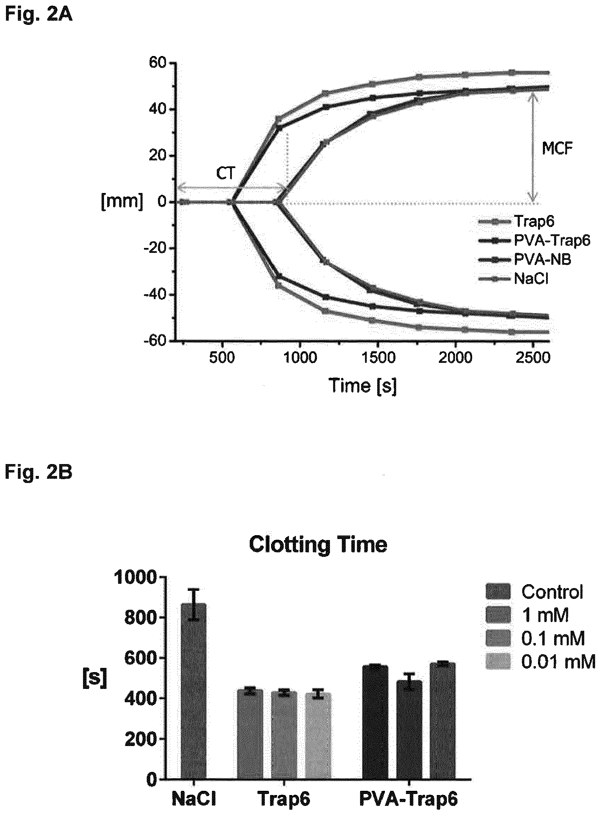

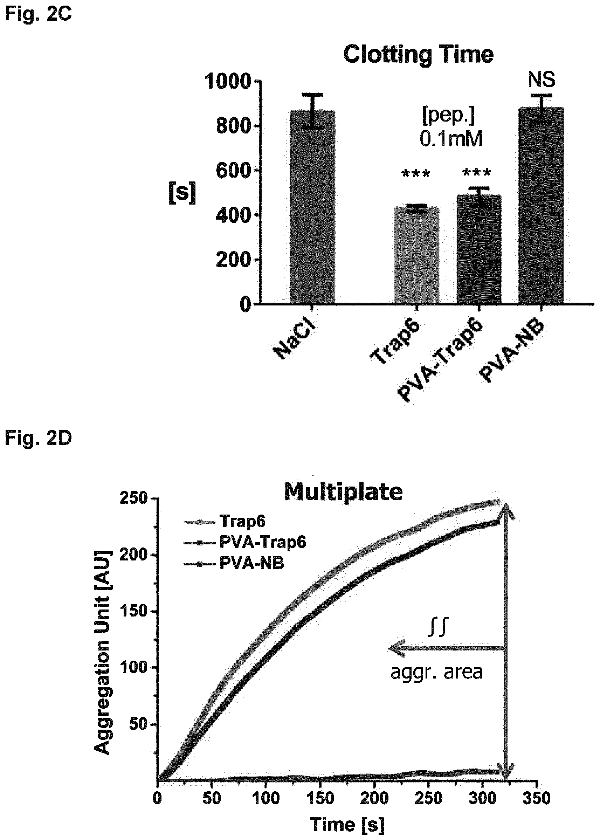

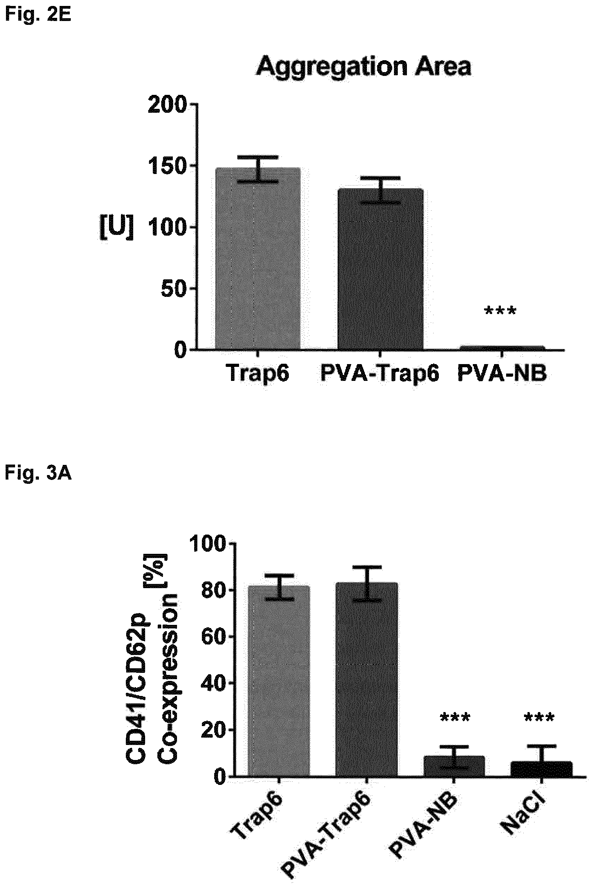

Fig. 2 shows (A)-(C) ROTEM characterization of the coagulation process of whole blood in response to the investigated materials (CT: clotting time in seconds, i.e. the latency until the clot reaches a firmness of 2 mm; MCF: maximum clot firmness in mm). (A) Plotted ROTEM curves showing the coagulation process of whole blood in response to TRAP6 (0.1 mM), PVA-TRAP6 (0.1 mM TRAP6-), PVA-NB, and 0.9% NaCl control; (B) Influence of unconjugated- and conjugated-TRAP6 (PVA-TRAP6) at varying dosage (0.01, 0.1, 1 mM) on CT; and (C) comparative analysis on CT between TRAP6, PVA-TRAP6, and PVA-NB at optimal TRAP6- concentration (0.1 mM). (D) Multiplate analysis of platelet function in response to TRAP6 (0.1 mM), PVA-TRAP6 (0.1 mM TRAP6), and PVA-NB; each measurement was performed in duplicate. (E) Comparison of the key parameter in Multiplate: aggregation area in Units.

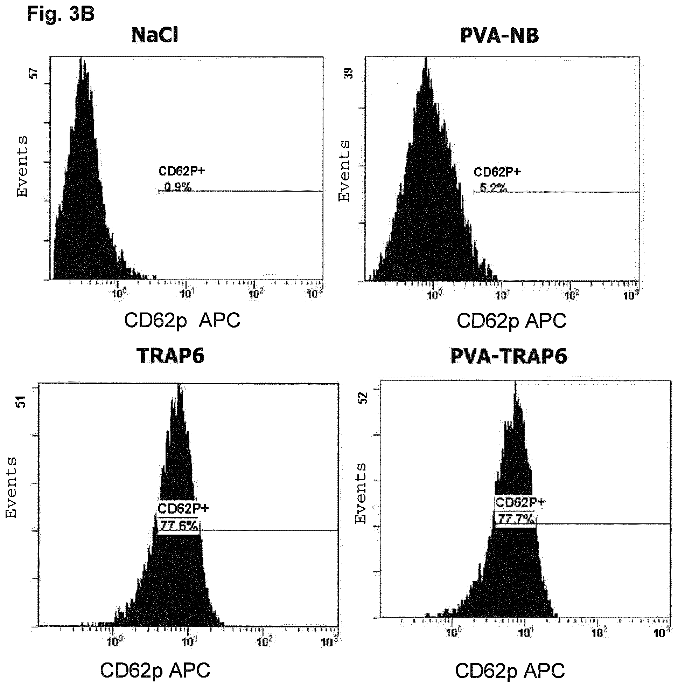

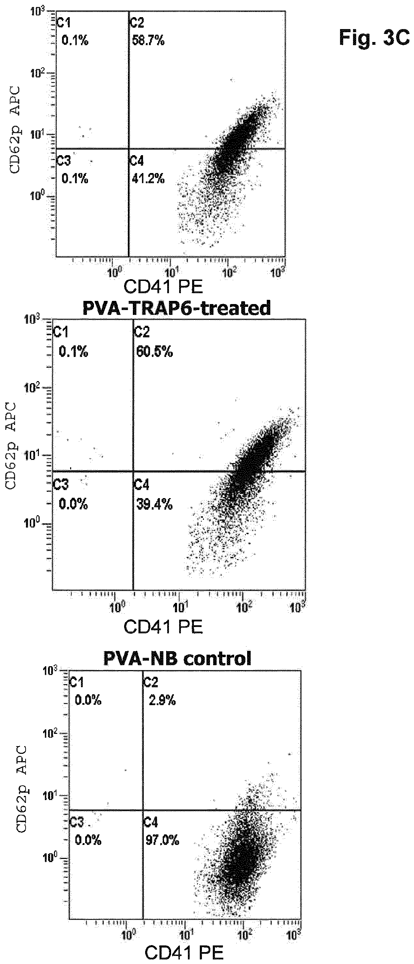

Fig. 3 shows (A) FACS analysis of TRAP6-mediated platelet activation measured by determination of CD62p/CD42 co-expression after 15 min incubation. Experiments were run in duplicate, data are presented as percentage of platelets positive for both CD62p and CD41 epitopes ±SD. (B) Histogram plots showing the value of the sample stained with the specific CD41 PE and CD62p APC antibodies. (C) Representative dot-plots for the expression of CD62p and CD41 of a TRAP6 treated sample, a PVA-TRAP6 treated sample, and a PVA-NB treated control after 15 min incubation.

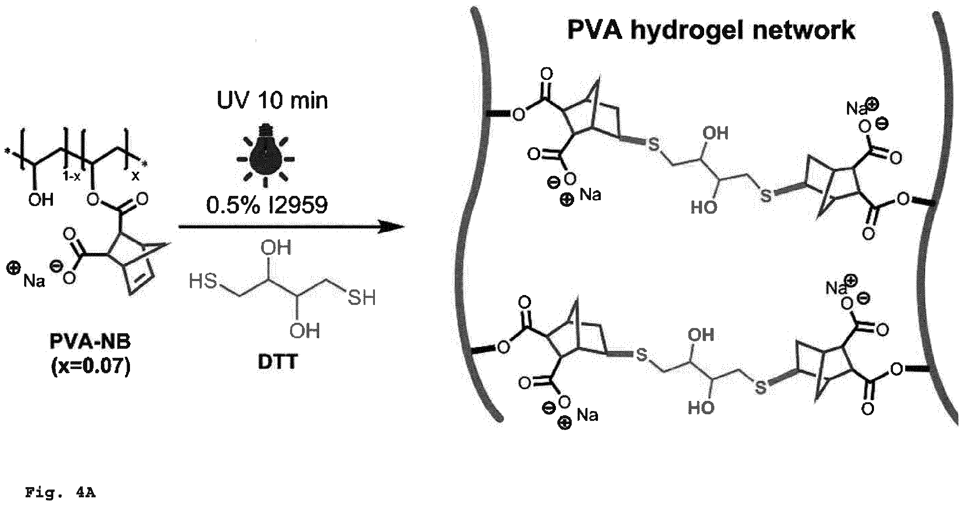

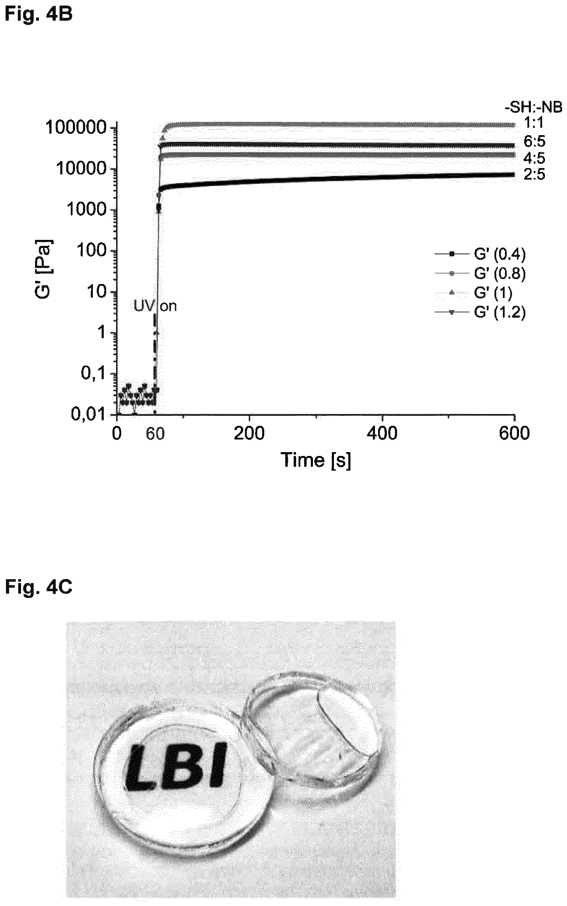

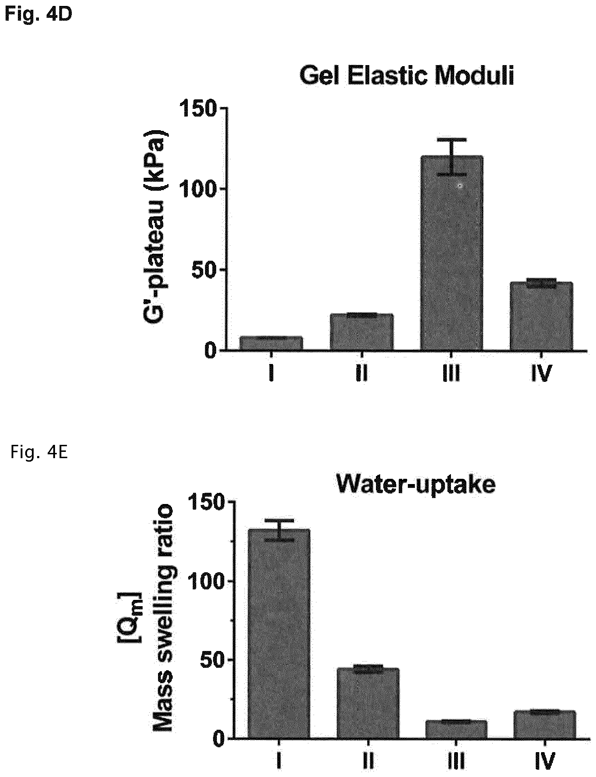

Fig. 4 shows (A) schematic showing the preparation of PVA-NB hydrogels by UV-photocrosslinking of PVA-NB with dithiothreitol (DTT) through radical-mediated photo-click chemistry. (B) Mechanical characterization of PVA hydrogels with varying thiol-to-NB ratios (0.4, 0.8, 1.0 and 1.2) using *in situ* oscillatory photo-rheometry: G'- gel storage moduli, 10% PVA-NB, 0.5% 12959, 60 s delay, light intensity: 20 mW cm⁻²; 50 µm gap thickness, 10% strain, 10 Hz. (C) Representatives of photopolymerized PVA-NB hydrogel pellets (scale bar: 1 cm). (D) Influence of thiol-to-NB ratio (N) on the G'-plateau value: N=0.4 (I), 0.8 (II), 1.0 (III), 1.2 (IV). (E) Equilibrium mass swelling ratios of PVA-NB hydrogels (I-IV) after swelling in PBS for 48 h.

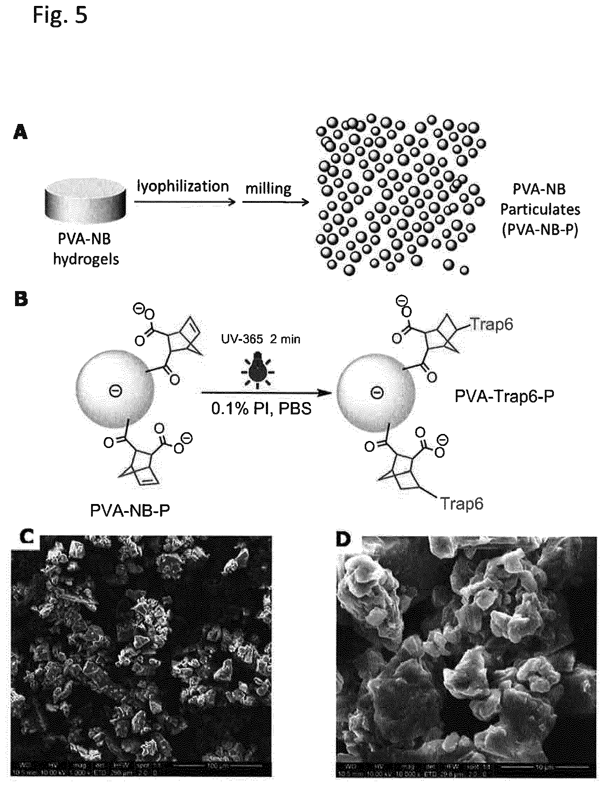

Fig. 5 shows (A) schematics of the preparation of PVA hydrogel (I, -SH:-NB=0.4) particulates (PVA-NB-P) by sequential lyophilization and cryo-milling,; (B) schematics of the surface functionalization of PVA-NB-P with cysteine-containing TRAP6 peptide via light-triggered thiol-NB conjugation, -SH:-NB=1.2, 0.1% PI (Li-TPO) in PBS, 20 mW cm⁻²; (C, D) SEM images of PVA-TRAP6-P, scale bars: 100 µm (C), 10 µm (D).

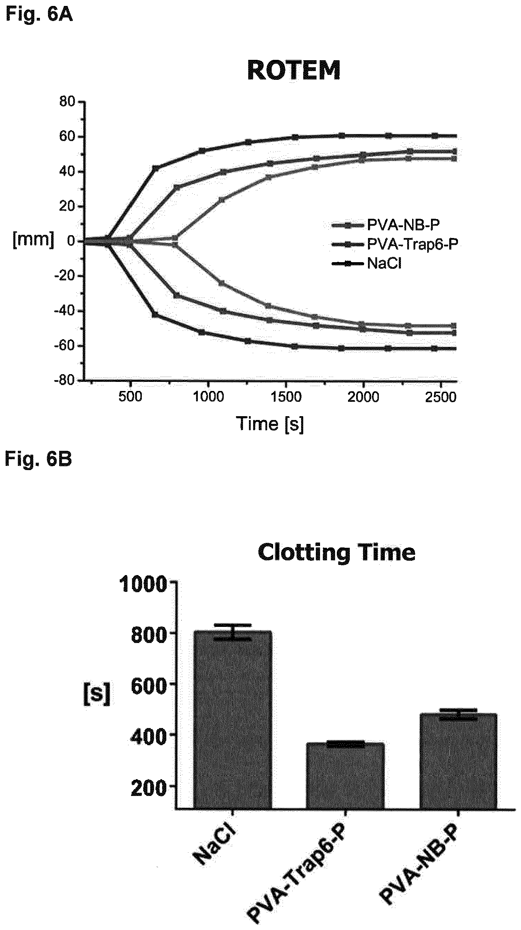

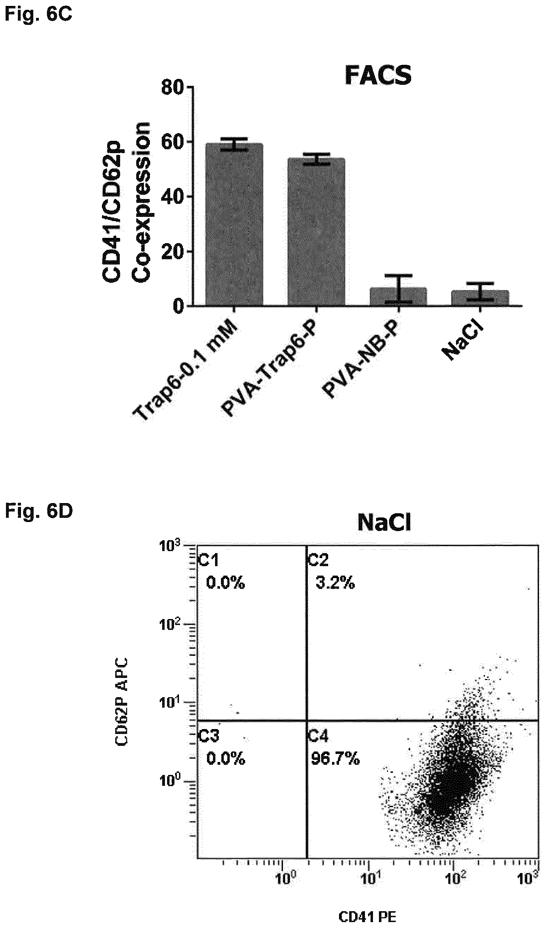

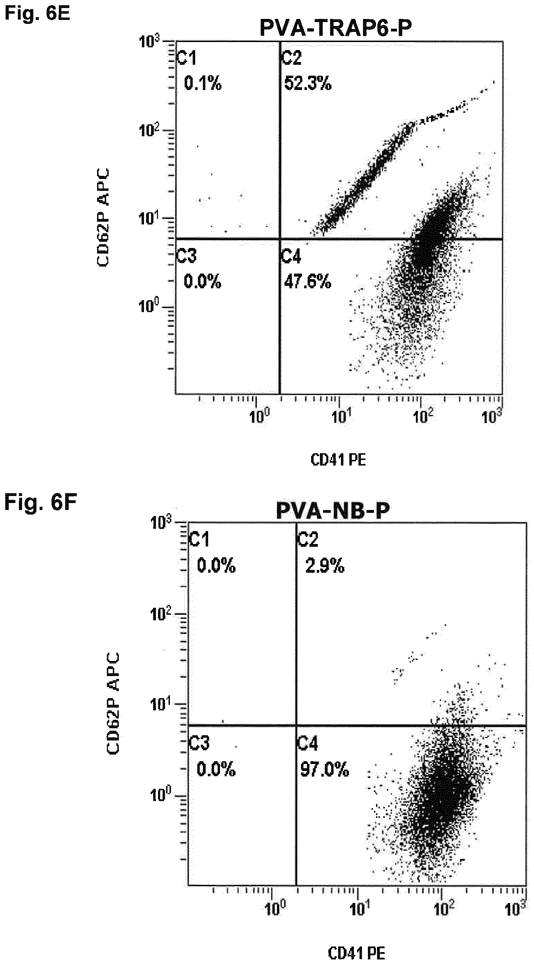

Fig. 6 shows (A)-(B) ROTEM characterization of the coagulation process of whole blood in response to the suspension of PVA-NB-P and PVA-TRAP6-P (10 wt% in saline). (A) Plotted ROTEM curves showing the coagulation process of whole blood in response to PVA-NB-P and PVA-TRAP6-P suspensions; (B) comparative analysis on the CT between PVA-NB-P, PVA-TRAP6, and NaCl (control). (C) - (F) FACS analysis of particulated polymers. (C) FACS analysis of TRAP6-mediated platelet activation measured by determination of CD62p/CD41 co-expression after 15 min incubation. Experiments were repeated twice using blood samples from different donors (n=2), and data are presented as percentage of platelets positive for both CD62p and CD41 epitopes ±SD. (D,E,F) Representative dot-plots for the co-expression of CD62p and CD41 of a PVA-NB-P treated sample and a PVA-TRAP6-P treated sample (positive control: 0.1 mM TRAP6, negative control: NaCl) after 15 min incubation.
Fig. 7 shows (A) molecular mechanism of protease activated receptor-1 (PAR-1) activation. (B) TRAP6- peptide motifs are covalently immobilized within synthetic polyvinyl alcohol (PVA) hydrogels, i.e. TRAP6-presenting hydrogels, which are capable of activating platelets in a highly localized manner.

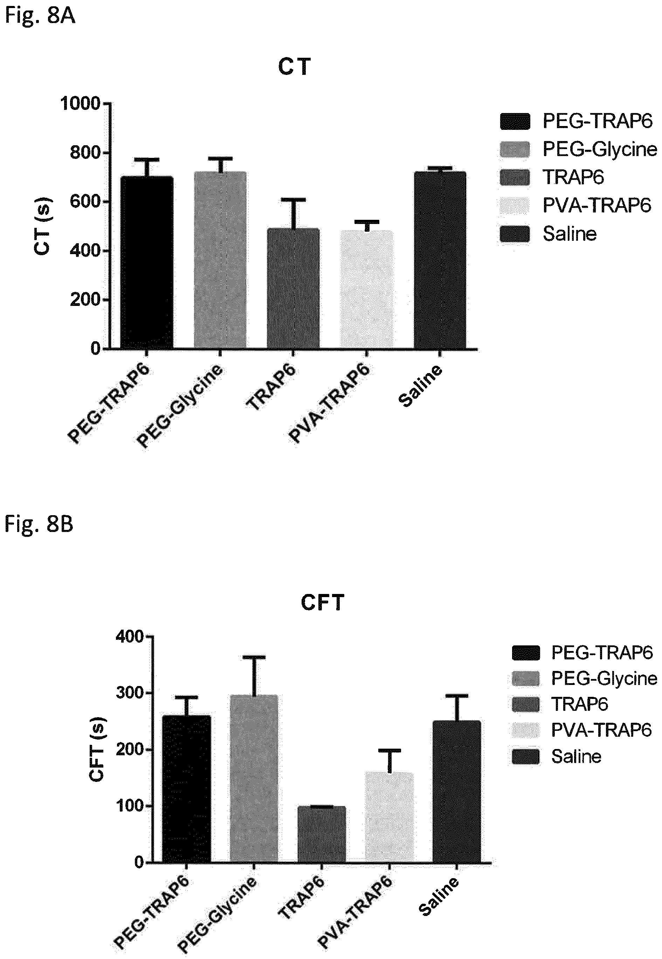

Fig. 8 shows the influence of TRAP6 (non-conjugated, 1 mM) and polymer-TRAP6 conjugates on blood coagulation measured by rotational thromboelastometry (ROTEM). A, clotting time (CT); B, clot formation time (CFT). PEG-TRAP6 was prepared by reacting PEG-10k-NHS with the N-terminus of TRAP6, while PVA-TRAP6 was prepared by reacting photo-clickable PVA norbornenes (22 kDa) with the cysteine residue in TRAP6 (SFLLRNPNC). PEG-10k-Glycine was used as the blank polymer control. All samples were measured in triplicates.

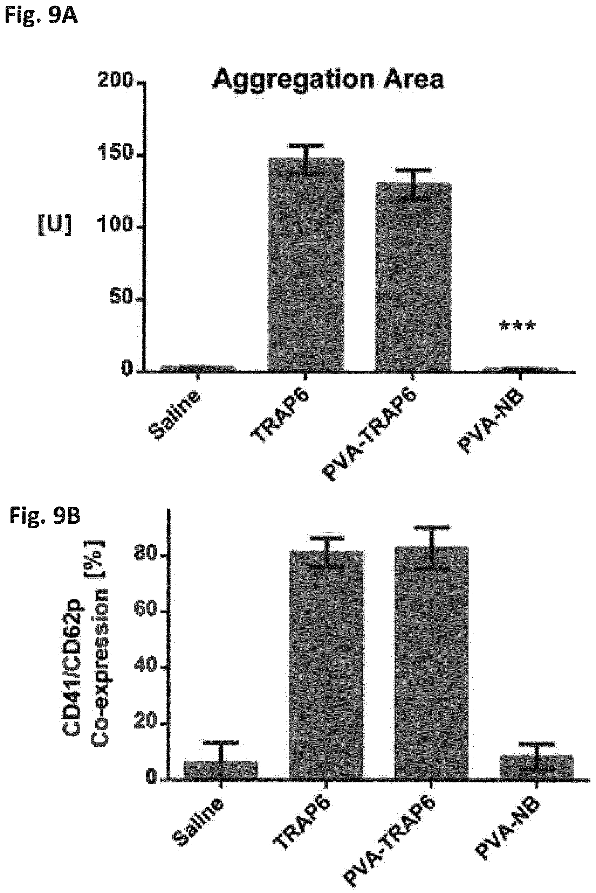

Fig. 9 shows experimental proof of retained bio-activity in PVA-TRAP6 (1 mM) in comparison to 1 mM TRAP6, 1 mM PVA-NB and saline. A, total platelet aggregation area measured by Multiplate-TRAP method; B, level of CD41/CD62P co-expression due to platelet activation measured by flow cytometry (FACS).

### Examples:

### Development of Synthetic Platelet-Activating Hydrogel Matrices to Induce Local Hemostasis

The present examples demonstrate the present invention by way of a water-soluble PVA-TRAP6 conjugate as model platelet-activating polymers as well as insoluble (crosslinked) PVA-TRAP6 hydrogel particulates (PVA-TRAP6-P) for safe and localized acceleration of hemostasis. In this work, it is demonstrated for the first time that TRAPs platelet-activating peptides, such as TRAP6, can be covalently immobilized in synthetic hydrogel matrices (Fig. 7B) for hemorrhage control. With this hemostatic material the hypothesis was tested that polymer-conjugated TRAP6 peptides can maintain their activity for platelet activation while accelerating hemostasis in a safe, localized manner and no systemic release of the platelet-activating agent can occur. The water-soluble PVA-TRAP6 conjugates was designed as model platelet-activating polymers as well as insoluble (crosslinked) PVA-TRAP6 hydrogel particulates (PVA-TRAP6-P) for safe and localized acceleration of hemostasis. These new polymer-peptide conjugates were prepared using highly efficient thiol-norbornene photo-click chemistry. The extent to which these materials could activate platelets was systematically characterized using rotational thromboelastography (ROTEM), platelet aggregation assay (Multiplate) and flow cytometry (FACS).

Several hemostatic strategies rely on the use of blood components such as fibrinogen and thrombin, which suffer from high cost and short shelf-life. In the present examples, a cost-effective synthetic biomaterial is developed for rapid local hemostasis. Instead of using thrombin, thrombin-receptor-agonist-peptide-6 (TRAP6) is covalently engineered in polyvinyl alcohol (PVA) hydrogels. Soluble PVA-TRAP6 was firstly prepared by covalent attachment of cysteine-containing TRAP6 onto the backbone of PVA-norbornenes (PVA-NB) through photo-conjugation. Cytotoxicity studies using C2C12 myoblasts indicated that PVA-NB and PVA-TRAP6 are nontoxic. Thromboelastography revealed that hemostatic activity of TRAP6 was retained in conjugated form, which was comparable to free TRAP6 solutions with equal concentrations. A 0.1% PVA-TRAP6 solution can shorten the clotting time (CT) to ∼45% of the physiological CT. High platelet-activating efficiency was further confirmed by platelet aggregation assay and FACS. For potential clinical applications, TRAP6-presenting hydrogel particulates (PVA-TRAP6-P) were developed for local platelet activation and hemostasis. PVA-TRAP6-P was prepared by biofunctionalization of photopolymerized PVA-NB hydrogel particulates (PVA-NB-P) with TRAP6. It was demonstrated that PVA-TRAP6-P can effectively shorten the CT to ∼50%. FACS showed that PVA-TRAP6-P can activate platelets to a comparable extent as soluble TRAP6 control. Altogether, PVA-TRAP6-P represents a promising class of biomaterials for safe hemostasis and wound healing.

### 1. Experimental Section

### 1.1. Materials and Reagents.

All reagents were purchased from Sigma-Aldrich and used as received unless otherwise noted.

### 1.2. Synthesis of PVA-Norbornene (PVA-NB).

In a three-neck flask, 10 g of PVA (22 kDa) and 20 mg of p-toluenesulfonic acid were dissolved in 250 mL of anhydrous DMSO at 60 °C for 1 h under argon atmosphere. In a second flask, under argon atmosphere 2 g of cis-5-norbornene-endo-2,3-dicarboxylic anhydride (0.1 eq. to -OH groups) was dissolved in 50 mL of anhydrous DMSO. The obtained solution was added dropwise into the first flask containing PVA. The reaction was maintained at 50 °C for 12 h. After reaction, the crude product was purified by dialysis against 10 mM NaHCO₃ solution for 24 h and subsequently against deionized (DI) water for 12 h. After lyophilization, PVA-NB was obtained as colorless solid in 95% yield. ¹H-NMR (D₂O) : 8 (ppm) : 6.2 (2H, s, -CH=CH-), 3.3 (2H, s, - C=CCH-CH-), 3.1 (2H, s, -C=C-CH-CH-), 1.3 (2H, s, -CH2-).
Degree of substitution (DS): 7.5%.

### 1.3. Synthesis of PVA-TRAP6 conjugates.

PVA-TRAP6 was prepared by covalent attachment of a cysteine-containing TRAP6 peptide (N-C: SFLLRNPNC (SEQ ID NO:15), China Peptide Co.) onto the backbone of PVA-NB through thiol-ene photo-click conjugation. Specifically, 60 mg of PVA-NB was dissolved in PBS solution of 0.5% Irgacure 2959 (I2959, BASF) to give a final macromer concentration of 5%. To this solution, 100 mg of TRAP6-Cys peptide (1.2 Eq. to NB groups) was added. The obtained solution was stirred under argon and irradiated with filtered UV-light (320-500 nm) for 300 s at 20 mW cm⁻². The UV-light was guided from an Omnicure S2000 lamp.

### 1.4. Preparation of PVA-NB hydrogels.

PVA-NB (DS-7.5%) was dissolved in 0.5% 12959 solution, achieving a final concentration 10 %. Then, aliquots of this solution was mixed with appropriate amount of dithiothreitol (DTT), providing -SH/-NB ratios as 0.4 (I), 0.8 (II), 1.0, and 1.2 (III), respectively. Hydrogel pellets were prepared by photopolymerization in a multi-well PDMS mold (well diameter: 6 mm). Specifically, 200 µL of macromer solutions were pipetted between two glass coverslips separated by the PDMS mold (thickness: 1.5 mm) and then exposed to filtered UV light (20 mW cm⁻²) for 600 s. Pellets were detached from the slides and washed with sterile PBS.

### 1.5. Preparation of PVA-NB hydrogel particulates (PVA-NB-P).

Hydrogel precursor solutions (I-III) were prepared as aforementioned and photopolymerized at same conditions except using a 10 mL cylindrical glass vial as the mold. After photopolymerization, the hydrogel cylinders were transferred into a 100 mL beaker and washed with PBS (2 changes) for 12 h in order to remove unreacted polymer and PI. Afterward, the swollen hydrogels were frozen with liquid N₂ and lyophilized. Finally, the dry PVA-NB matrix was grinded into fine powders using a RETSCH Cryomill RS232.

### 1.6. Preparation of TRAP6-presenting PVA hydrogel particulates (PVA-TRAP6-P).

PVA-TRAP6-P was prepared by covalent attachment of TRAP6-Cys peptide onto the residual NB groups on PVA-NB-P. Specifically, 100 mg of PVA-NB was dispersed in PBS solution of 0.1% visible light PI (LAP)^{[19]} to give a final polymer content of 5%. To this suspension, specific amounts of TRAP6-Cys peptide (1.2 Eq. to NB groups) were added. The obtained suspension was stirred under argon and irradiated with UV-light (365 nm) for 300 s at 20 mW cm⁻². The UV-light was guided from an Omnicure LX400 LED lamp.

### 1.7. Photo-rheometry.

Photo-rheometry was performed on a modular photo-rheometer (Anton Paar MCR-302) as previously reported.^{[27]} Specifically, MCR302 was integrated with filtered UV-light (320-500 nm) from a light guide (Omnicure S2000) to the bottom of the glass plate. Specifically, plate-to-plate oscillatory photo-rheometry was applied for real-time monitoring of the curing kinetics of hydrogel formulations during photopolymerization. Light intensity at the plate surface was ∼20 mW·cm⁻² as determined by an Ocean Optics USB 2000+ spectrometer. Both storage moduli (G') and loss moduli (G") of the samples could be monitored as a function of irradiation time. Gel point was determined in the vicinity of the G' and G" crossover.

### 1.8. Water-uptake.

Mass swelling ratios of PVA-NB hydrogels were tested using a generic protocol. ^{[28]} Hydrogel pellets (n = 3) were prepared as aforementioned and allowed to swell in DI H₂O for 24 h at room temperature. The wet pellets were weighed to determine the equilibrium swollen mass (Mₛ) and then lyophilized to obtain the dry weight (M_{d}). The equilibrium mass swelling ratio (Qₘ) was calculated as M_{s/}M_{d}.

### 1.9. MTT assay.

Cytotoxicities of PVA, PVA-NB and PVA-TRAP6 macromer solutions were evaluated via MTT assay. C2C12 cells were cultured in Dulbecco's Modified Eagles Medium (DMEM) supplemented with 5% fetal calf serum (FCS), 1% L-Glutamine, 1% Penicillin/Streptomycin (all from Sigma-Aldrich, Austria). Macromer solutions with three concentrations (5%, 1% and 0.1%) were prepared in DMEM culture medium. C2C12 cells were then seeded in a 96-well plate at a density of 5 × 10³ cells per well in 200 µL of culture medium. After 24 h incubation (37 °C, 5% CO₂), 100 µL of the respective macromer solutions were added to the cells in triplicates. After 24 h incubation, cells were washed twice with sterile PBS before the addition of 100 µL thiazolyl blue tetrazolium bromide (MTT) working solution (5 mg/mL in PBS). After 1 h incubation, the liquid was discarded and 100 µl of DMSO was added to dissolve the formazan crystals. Finally, the absorbance was measured at 540 nm using a microplate reader.

### 1.10. Rotational thromboelastometry (ROTEM).

ROTEM (TEM Innovation, Germany) was applied to monitor the interactions of platelet-activating polymers and whole blood over time. ROTEM system contains an oscillating sensor pin that is immersed in a temperature-controlled cuvette containing the blood sample. Four measurements can be performed in parallel in the same device. Generally, coagulation of the citrated blood sample is initiated by re-calcification. The formation kinetics of a fibrin clot could be monitored mechanically and calculated by an integrated computer to the typical curves and numerical parameters.

Blood was collected from three un-medicated and healthy donors using minimal stasis from an antecubital vein through a 21-gauge needle. After discarding the first 3 ml, blood was collected in 3.5 ml tubes (Vacuette; Greiner Bio-One) containing 0.3 ml buffered 3.2 % sodium citrate. The samples were kept on a pre-warming stage at 37°C for at least 10 min prior to analysis and were processed within 3 h. ROTEM analysis of the whole blood sample was initiated by recalcification with the addition of 20 µl of CaCl₂ (star-TEM®, 200 mM). Polymer solutions and/or polymer suspensions were added directly to the cuvette immediately after recalcification of the citrated blood and mixed by gently pipetting up and down as previously reported. ^{[21]} The final reaction volume per ROTEM cuvette was 370 µl, consisting of 300 µl citrated whole blood, 20 µl CaCl₂ and 50 µl polymer solution or polymer suspension.

The following ROTEM parameters were calculated from the signal and included in the statistical analysis: clotting time (CT) in seconds (sec), i.e. the latency until the clot reaches a firmness of 2 mm, which is a measure of initial fibrin formation; clot formation time (CFT) in sec, i.e. the time from CT until the clot reaches a firmness of 20 mm, which indicates platelet function and fibrinogen quality; α-angle, the angle (°) between the x-axis and the tangent of the forming curve starting from CT point, which is comparable to CFT; maximum clot firmness (MCF) in mm, the maximum amplitude of the curve, which indicates the absolute strength of the clot; and A30 (mm), i.e. the clot firmness after 30 min.

### 1.11. Multiplate Analysis.

The principle of Multiplate test is based on the fact that platelets become sticky upon activation, and thus prone to adhere and aggregate on the metal sensor wires in the Multiplate test cuvette. The sensor wires are made of highly conductive copper, which is silver-coated. As activated platelets adhere and aggregate on the sensor wires, the electrical resistance between the wires rises, which can be monitored in real time. For each measurement, 300 µL of saline and 300 µL of hirudinized whole blood was added sequentially to a Multiplate cuvette. After 3 min incubation at 37 °C, 20 µL of sample solution was added and at the same time the program started to collect signals. The Multiplate device allows 4 measurments in parallel. Each measurement was performed for 6 min and in duplicate.

### 1.12. FACS Analysis.

The blood of two unmedicated and healthy donors was collected and stabilized by sodium citrate. To 110 µL of whole blood (WB), 20 µL of PVA-TRAP6 solution (30 mg mL⁻¹ and 3 mg mL⁻¹ to obtain final TRAP6 concentrations in the WB of 2 mM and 0.2 mM) were added and incubated for 15 min at 37 °C. Twenty microliter of TRAP6-C solution (14 mg mL⁻¹) and/or 20 µL of NaCl solution (0.9%) were added as the positive control and the negative control, respectively. The incubated mixture was mixed with 300 µL of 1% paraformaldehyde for 15 min at room temperature. After washing and centrifugation a double immuno-staining was performed using 20 µL of phycoerythrin (PE) labeled monoclonal antibody directed against CD41 and 20 µL of allophycocyanin (APC) labeled monoclonal antibody directed against CD62P. Isotype controls were incubated with mouse IgG antibodies conjugated with APC or PE dye, respectively (all antibodies were purchased from BD Biosciences, Heidelberg, Germany). After 30 min incubation at room temperature the activation state of the platelets was determined using fluorescence flow cytometry. Expression of the platelet activation marker CD62P and the constitutively present platelet marker CD41 were detected using a Beckmann Coulter Cytomics FC-500 equipped with Uniphase Argon ion laser, 488 nm, 20 mW output. Overall 100 000 platelets were measured per sample and analyzed with the Cytomics CXP software. The experiment was repeated twice.

### 1.13. Statistics.

All error bars indicate the standard deviation. The statistical significance was determined by student's t-test, where '*', '**', '***' indicate P < 0.05, P < 0.01, P < 0.001, respectively.

### 2. Results & Discussion

### 2.1. Materials Design and Synthesis

In this study, a linear synthetic polymer polyvinyl alcohol (PVA) was selected as the substrate for covalent immobilization of the potent platelet-activating peptide (TRAP6) due to the following reasons. First, PVAs are FDA-approved polymers with superior cost-efficacy and cytocompatibility, therefore intriguing for many biomedical applications. ^{[17]} Second, the existence of a high number of hydroxyl groups in PVA offers significant freedom for tunable functionalization and presentation of bioactive ligands, which is not available with other synthetic substrates such as arm-dependent polyethylene glycol (PEG).

To introduce TRAP6 peptides onto PVA, we chose the robust thiol-ene photo-click chemistry as the conjugation approach. On one hand, norbornene group was selected as the ene functionality due to its ultrahigh reactivity towards thiol-ene reaction as well as low cytotoxicity. ^{[18, 19]} On the other hand, we engineered a cysteine moiety with a free thiol group into the C-terminus of a TRAP6 peptide sequence (SFLLRNPNC), since it is accepted that the N-terminus of TRAP6 sequence is critical for its ability to activate platelets. ^{[15]}

PVA-NB was synthesized through a facile esterification reaction between PVA and norbornene anhydride for 12 h at 50 °C in DMSO (Figure 1A). One notable advantage of this modification approach is that after modification a high number of carboxylate groups could be neutralized into the sodium salt form to provide the products with good water-solubility. The crude products were purified by sequential dialysis against 10 mM NaHCO₃ for neutralization and later on against H₂O, and finally lyophilized (>95% yield). To confirm the synthesis, PVA-NB was analyzed using ¹H-NMR in comparison with unmodified PVA. As shown in Figure 1B (bottom), the spectrum of unmodified PVA represents two major peaks at 4.0 ppm and 1.6 ppm, which are corresponding to the -CH- and methylene groups, respectively. The spectrum of PVA-NB (Figure 1B, middle) shows new peaks at 6.2 ppm (s, 2H, - CH=CH-), 3.3 ppm (s, 2H, -C=C-CH-CH-), 3.1 ppm (s, 2H, -C=C-CH-CH-) and 1.3 ppm (s, 2H, -CH2-), respectively. The degree of substitution (DS) of PVA-NB was determined by comparing the integral values corresponding to signals (a, d, f). By changing either the stoichiometry between the reactants or reaction time, it was feasible to precisely control the DS in a wide range from 5%-50% (Table S1). Since PVA (22 kDa) is a linear polymer consisting of ∼500 repeating units, we selected PVA-NB with the lowest DS (DS-7%) as the precursor, providing ∼35 reaction sites for photo-conjugation with cysteine-containing peptide.

PVA-TRAP6 conjugates were prepared by photo-conjugation of cysteine-containing TRAP6 peptide with the NB groups of PVA-NB in PBS solution of 12959 as photoinitiator (PI). To confirm the conjugation efficiency, NMR model reactions were firstly performed in D₂O. Based on the NMR reaction, Figure 1B (Top) represents the spectrum of PVA-TRAP6 conjugates. The significant decrease of NB proton signals (a) at 6.2 ppm indicates the success of conjugation. Besides, the spectrum of PVA-TRAP6 also shows a new peak at 7.4 ppm, corresponding to the aromatic protons of phenylalanine (Phe or F) moieties in the TRAP6 sequence.

### 2.2. In-vitro Cytotoxicity

To prove the applicability of the prepared materials for biomedical applications, the *in vitro* biocompatibility of PVA, PVA-NB and PVA-TRAP6 solutions was investigated by MTT assay using C2C12 myoblasts. MTT assay showed that PVA and PVA-NB (Figure 1C) solutions were non-toxic at varying concentrations (0.1, 0.5, 1 %) after 24h and 48h incubation. For the PVA-TRAP6 conjugates, the metabolic activity of C2C12 cells **(****Figure 1D****)** after 24h incubation was significantly increased when mixed with 1% PVA-TRAP6 (P < 0.001), while not increased for 0.5% and 0.1% PVA-TRAP6. After 48h incubation, the metabolic activity for all three concentrations was significantly increased compared to the control (P < 0.001). Several studies by other groups have shown that PAR-1 activating peptide such as TRAP6 can stimulate cytokine release from different cell types, including human gingival fibroblasts, endothelial cells, intestinal epithelial cells, and human muscle myoblasts. ^{[20 a,b,c]} Therefore, we assume that the increased metabolic activity of C2C12 myoblasts during MTT assay is attributed to TRAP6-induced PAR-1 activation. Considering that a 1% PVA-TRAP6 solution gives a TRAP6-concentration of 5 mM whereas the effective TRAP6- concentration for platelet activation is in the range of 5-100 µM, ^{[12]} the toxicity results suggest that PVA-TRAP6 are cytocompatible materials within its effective range.

### 2.3. Hemostatic Activity

### 2.3.1. Thromboelastometry

We next studied the hemostatic efficacy of PVA-TRAP6 in comparison with TRAP6 and PVA-NB using rotational thromboelastometry (ROTEM), ^{[21, 22]} which is a clinical diagnostic tool allowing *in situ* characterization of viscoelastic properties of blood clot during coagulation. Figure 2A shows the plotted ROTEM curves of the studied samples that were mixed with recalcified whole blood. Clotting time (CT) refers to the latency until the clot reaches a firmness of 2 mm while maximum clot firmness (MCF) refers to the maximum amplitude of the curve, which indicates the absolute strength of the clot.

From the ROTEM results, it was observed that the CT of PVA-TRAP6 at 0.1 mM was very comparable to that of TRAP6 at 0.1 mM while the MCF of PVA-TRAP6 was relatively less than that of TRAP6 control. The lower MCF might be attributed to the decreased accessibility of TRAP6- to platelets after conjugation in PVA-TRAP6, which is a macromolecular conjugate (65 kDa) and significantly larger than TRAP6 (1 kDa). In comparison, the PVA-NB control **(****Figure 2C****)** showed a curve very similar to the physiological curve (NaCl control), showing no hemostatic activity of the PVA-NB backbone.

To test whether the hemostatic activity of PVA-TRAP6 is dose-dependent, we tested PVA-TRAP6 solutions in comparison with TRAP6 solutions at three peptide concentrations (0.01, 0.1, 1 mM) in ROTEM (Figure 2B). It was found that the optimal hemostatic concentration for PVA-TRAP6 was 0.1 mM while there was no significant dose influences for TRAP6 control in the chosen range. This may again imply the influence of differential molecular structure in PVA-TRAP6 and TRAP6 peptide on the saturation level of TRAP6 for platelet activation.

### 2.3.2. Multiplate Analysis

In order to quantify the extent of platelet activation, we utilized Multiplate assay to investigate the influence of PVA-TRAP6, TRAP6 and PVA-NB on platelet aggregation. The principle of this method is based on the fact that platelets become sticky upon activation, and thus prone to adhere and aggregate on the metal sensor wires in the Multiplate test cuvette. As activated platelets adhere and aggregate on the sensor wires, the electrical resistance between the wires rises, which can be continuously monitored. A typical Multiplate curve (Figure 2D) represents the accumulation of electronic signals corresponding to the extent of platelet aggregation. One key parameter of Multiplate assay is aggregation area (in Units), i.e. the area underneath the aggregation curve. It was observed that PVA-TRAP6 (0.1 mM) induced an aggregation curve (Figure 2D) that was comparable to that of TRAP6 (0.1 mM), while the PVA-NB control displayed negligible capability of platelet aggregation. The aggregation area value (Figure 2E) for TRAP6 was 147 U whereas the area value for PVA-TRAP6 and PVA-NB was 130 U and 2 U (p<0.001), respectively. Together, Multiplate assay proved that PVA-TRAP6 presented high efficiency for platelet activation while the substrate (PVA-NB) did not.

### 2.3.2. FACS of the Soluble System

We next utilized flow cytometry (FACS) to quantify the extent of platelet activation. Platelets can be distinguished from other blood cells by the constitutive expression of the surface antigen CD41, which recognizes the platelet membrane glycoprotein GpIIb which is non-covalently associated with GpIIIa (the integrin beta 3 chain) to form the GpIIb/IIIa complex. Importantly, the CD62p (P-selectin) membrane glycoprotein is exclusively expressed on activated platelets. The CD62p marker was used to identify the extent of activation in human platelets after incubation with the studied materials (PVA-TRAP6, TRAP6, PVA-NB, and NaCl). The measurement of the CD41/CD62p co-expression (Figure 3 A-C) in blood samples treated with these materials for 15 min revealed that there was significant effect (∼80%) of PVA-TRAP6 (0.1 mM) on the CD62p expression in CD41 positive cells. The percentage of activated platelet phenotype in terms of CD62p positive cells stayed at the same level of the control sample treated with TRAP6 (0.1 mM). By contrast, there was no significant effect (<10%) of PVA-NB on the CD62p expression in CD41 positive cells, which was at the same level of the negative control samples treated with 0.9% NaCl. In all, FACS analysis further confirmed the high efficiency of PVA-TRAP6 for platelet activation.

### 2.4. Preparation and Characterization of PVA Hydrogels

Since platelet-activating PVA-TRAP6 in soluble form or in releasable form, such as in WO 96/40033 A1 has the potential to cause thrombotic risks in the circulation, we further developed an insoluble PVA-TRAP6 system for localized hemostasis whereby TRAP6 peptide were covalently immobilized in photocrosslinked PVA hydrogel matrices. We selected radical-mediated thiol-NB photopolymerization as the approach to create PVA hydrogel matrices (Figure 4A). In contrast to conventional crosslinking chemistry of (meth)acrylates, thiol-NB photopolymerization offers several advantages, including robust kinetics, excellent spatiotemporal control and cytocompatible conditions. ^{[19, 23]} For instance, PEG-based thiol-NB hydrogels have enabled *in situ* photo-encapsulation of mammalian cells with high viability (>90 %). ^{[19]} In this study, PVA-NB macromers in combination with a model crosslinker (dithiothreitol, DTT) were photopolymerized under UV irradiation in the presence of 12959 as a water-soluble and biocompatible PI.

### 2.4.1.Photo-rheometry

We utilized *in situ* photo-rheometry to test the photo-reactivity and mechanical properties of PVA hydrogels. It was hypothesized that the chemo-physical properties of PVA hydrogels could be easily adjusted by tuning the thiol to NB ratio. Four PVA-NB/DTT formulations with equal macromer content (10 %) but varying thiol to NB ratios (0.4, 0.8, 1.0, 1.2) were screened in photo-rheometry (Figure 4B). After a 60s blank period (no UV), upon UV irradiation the storage moduli (G') of PVA hydrogels increased to different extents (8-120 kPa) in seconds until reaching a G'-plateau. It was found that all of the photopolymerized PVA hydrogels were totally transparent (Figure 4C). By increasing the thiol to NB ratio from 0.4 to 1.2, the G'-plateau values (Figure 4D) changed from 8, 22, 120 to 45 kPa, respectively. The highest G'-plateau value was obtained for hydrogel (III) whereby the thiol to NB ratio was 1:1, indicating the highest degree of crosslinking. Notably, these G'-plateau values from photo-rheometry measurements can only represent the temporal storage moduli of PVA hydrogels in the pre-swollen state, as the swelling process could affect the storage moduli of the hydrogels. ^{[24]} Further investigation into the mechanical properties of swollen PVA hydrogels is warranted by using alternative approaches such as AFM Nanoindentation.

### 2.4.2. Water-uptake

We further analyzed the water-uptake properties of PVA hydrogels (I-IV). Photopolymerized PVA hydrogel pellets were soaked in PBS for 48h to reach an equilibrium wet weight (m_{wet}), which was compared to the polymer dry weight (m_{dry}) after lyophilization and give the equilibrium mass swelling ratio (Qₘ). As shown in Figure 4E, the Qₘ values of hydrogels (I-IV) changed from 130, 45, 10 to 17. In combination with the G'-plateau values, these data suggest that the highest crosslinking degree was obtained when the thiol to NB ratio was 1:1 (III). This observation correlates with previous studies on PEG-based thiol-NB hydrogels by other groups. ^{[19, 25]} For instance, Lin et al. demonstrated that thiol-NB photopolymerized PEG hydrogels are hydrolytically degradable due to the presence of ester bonds. ^{[25]} The degradation rate was dependent on the gel crosslinking density, which was dictated by thiol to NB ratio and macromer content. Since presented PVA hydrogels also possess a number of ester linkages, we anticipate that these hydrogels are hydrolytically degradable. Besides DTT, alternative di-cysteine protease-sensitive peptides can also be used as enzymatically cleavable crosslinker in order to foster cellular remodelling and wound healing. Nevertheless, further investigation into the degradation behavior of presented PVA hydrogels *in vitro* and *in vivo* is needed.

### 2.5. Biofunctionalization

### 2.5.1. Preparation of TRAP6-Functionalized Hydrogel Particulates

In order to prepare appropriate hydrogel matrices for TRAP6-functionalization, photopolymerized PVA hydrogels (I, -SH:-NB=0.4) were sequentially lyophilized and cryo-milled into fine particulates (Figure 5A). Since excessive NB groups were present after photopolymerization, these residual groups were exploited for photo-click conjugation (Figure 5B) with cysteine-containing TRAP6 peptide. SEM analysis (Figure 5C) revealed that the length scale of PVA-TRAP6-P was in the range of 5-50 µm. The partial agglomeration of PVA-TRAP6-P was presumably due to the charge effects of NB groups.

### 2.5.2. ROTEM Analysis

We tested the hemostatic ability of PVA-TRAP6-P in comparison with PVA-NB-P in ROTEM. Prior to test, these particulates were carefully mixed with saline to form injectable slurries. As shown in Figure 6 A-B, the addition of PVA-TRAP6-P into whole blood induced a significant decrease of CT to ∼50% of the physiological CT. Interestingly, the PVA-NB-P control also induced a decrease of CT to ∼70%. Since negatively charged surfaces are known to contribute coagulation (i.e. the intrinsic pathway), ^{[26]} we suppose that the observed hemostatic activity of PVA-NB-P is due to the charge effects of NB groups.

### 2.5.3. FACS Analysis

In order to quantify the ability of these particulated materials to activate platelets, we analyzed whole blood samples that were pre-incubated with PVA-TRAP6-P and/or PVA-NB-P in FACS. FACS analysis (Figure 6 C-F) revealed that the percentage of activated platelets (CD41⁺/CD62p⁺) for PVA-TRAP6-P was as high as ∼55%, which was comparable to the positive control (0.1mM TRAP6). By contrast, blood samples incubated with PVA-NB-P only exhibited a minimal amount of activated platelets (<10%). These results show that TRAP6-presenting hydrogel matrices (PVA-TRAP6-P) present good potency of activating platelets in a localized manner.

### 2.6. Comparison of conventional immobilization techniques with coupling techniques preserving activity of the peptidic thrombin receptor activating agent

To show the importance of diligently choosing the suitable immobilization technique, a comparison of conventional peptide immobilization methods (e.g. NHS conjugation) and methods which have been selected in the course of the present invention, such as the photo-click conjugation, was performed for the process of covalent binding of the TRAP6 peptide to polymer substrates. It turned out that conventional peptide immobilization methods lead to the loss of its bio-activity. This results in a lack of thrombin receptor activation and to no induction of blood coagulation, making the resultant materials not useful for local hemostasis.

To prepare polymer-TRAP6 via NHS conjugation, polyethylene glycol (10 kDa) with terminal NHS group (PEG-10k-NHS) was used as the polymer substrate. TRAP6 was linked to PEG-10k-NHS through reaction at the N-terminus, and unreacted NHS groups were blocked with glycine. Furthermore, PEG-10k-NHS reacted with excessive glycine was prepared as the negative control. After dialysis against distilled water and lyophilization, the conjugates were obtained in high yields and subsequently analyzed in standard rotational thromboelastometry (ROTEM) to study their effects on blood coagulation. In order to compare the bioactivity of polymer-TRAP6 conjugates prepared by different approaches, PVA-TRAP6 prepared by our claimed approach (i.e. photo-click conjugation) was included as the positive control. The samples consist of 1 mM TRAP6, 1 mM PEG-TRAP6, 1 mM PEG-Glycine, 1 mM PVA-TRAP6 and saline.

As shown in Figure 8, non-conjugated TRAP6 (1 mM) induced a significant decrease of clotting time (CT) and clot formation time (CFT) in comparison to the saline control. However, this effect was lost when TRAP6 was conjugated to the PEG10k using the conventional NHS conjugation procedure. Similar effects can also be observed for the PEG-Glycine conjugate control, showing minimal effects of the polymer backbone and the preparation procedure. Nevertheless, the PVA-TRAP6 conjugates prepared by photo-click conjugation could still significantly shorten the CT and CFT, showing that the bioactivity of TRAP6 is retained. In summary these data shows that the traditional NHS immobilization approach fails to retain the bioactivity of TRAP6. By contrast, the covalent immobilization approach based on thiol-norbornene photo-click conjugation can retain almost the full bio-functionality of the TRAP6 peptide.

To further demonstrate the ability of PVA-TRAP6 conjugates for platelet activation, we tested the samples in standard platelet aggregation assay (Multiplate) and flow cytometry (FACS). As shown in Figure 9A, the total platelet aggregation area of PVA-TRAP6 is comparable to that of TRAP6 at equal peptide concentration, while the PVA-NB substrate control and saline control show negligible level of platelet aggregation. Furthermore, FACS results (Figure 9B) confirm that PVA-TRAP6 conjugates prepared by photo-click conjugation can induce platelet activation (i.e. CD41/CD62P co-expression) to a very similar level as TRAP6 control (1 mM), while no significant platelet activation could be observed for PVA-NB and saline control. These findings prove that our conjugation approach indeed can retain the bioactivity of TRAP6, even when it is covalently immobilized to PVA.

In contrast to state-or-the-art approaches for peptide immobilization, the approach of the present invention is based on specific coupling techniques with no risk of side reactions with N- or C-terminus or acidic or basic amino acids by using e.g. bioorthogonal reactions, such as photo-click conjugation of cysteine-containing TRAP6 onto PVA norbornenes, which offers a very high degree of conjugation efficiency (>95%), site-specificity and modularity. The same conjugation approach is also applicable for other substrates bearing norbornene groups, such as naturally-derived molecules (gelatin, hyaluronan, alginate, etc.) and synthetic analogues such as PEG. In addition, the polymer-bound TRAP6 conjugates have lower probability to be internalized by blood cells than soluble TRAP6 peptide through PAR-1 receptor signaling, as the size of PVA substrates (hundreds of repeating units) is far larger than a short TRAP6 sequence. In all, the approach according to the present invention with coupling the thrombin receptor activating active agent with retained activity (e.g. by photo-click conjugation for TRAP6 peptide immobilization) provides significant practical values for local hemostasis as well as other medical applications.

### 3. Conclusion

In this work, we developed a synthetic hemostatic system that can efficiently activate platelets and accelerate hemostasis in a localized manner. The use of highly potent protease, thrombin, is avoided in this system. Instead, the thrombin receptor agonist peptide (TRAP) was covalently engineered on cytocompatible PVA hydrogels via highly efficient thiol-norbornene photo-conjugation. The presented TRAP6-functionalization approach is also applicable, but not restricted to other synthetic materials/hydrogels such as PEG as well as naturally-derived hydrogels such as gelatin and hyaluronic acid. From a biological point of view, activated platelets are known to release platelet-derived growth factors (PDNF), which regulate cell proliferation and play a significant role in blood vessel formation (angiogenesis). Therefore, we anticipate that these platelet-activating hydrogel matrices are versatile biomaterials not only for safe hemostasis but also for potential applications in tissue regeneration and wound healing.

### References

[1] H. B. Alam, D. Burris, J. A. DaCorta, P. Rhee, Mil Med 2005, 170, 63.
[2] W. D. Spotnitz, S. Burks, Transfusion 2008, 48, 1502; A. M. Behrens, M. J. Sikorski, P. Kofinas, Journal of biomedical materials research. Part A 2014, 102, 4182.
[3] (a) H. Redl, G. Schlag, in Fibrin Sealant in Operative Medicine, (Eds: G. Schlag, H. Redl), Springer Berlin Heidelberg, 1986, 13; (b) H. C. Hedrich, M. Simunek, S. Reisinger, J. Ferguson, H. Gulle, A. Goppelt, H. Redl, Journal of Biomedical Materials Research Part B: Applied Biomaterials 2012, 100B, 1507.
[4] (a) T. A. Ostomel, Q. Shi, G. D. Stucky, Journal of the American Chemical Society 2006, 128, 8384; (b) T. A. Ostomel, Q. Shi, P. K. Stoimenov, G. D. Stucky, Langmuir : the ACS journal of surfaces and colloids 2007, 23, 11233.
[5] (a) M. C. Oz, J. F. Rondinone, N. S. Shargill, Journal of cardiac surgery 2003, 18, 486; L. Y. Han, V. Schimp, J. C. Oh, P. T. Ramirez, International journal of gynecological cancer : official journal of the International Gynecological Cancer Society 2004, 14, 621; (b) R. Lemmer, M. Albrech, G. Bauer, The journal of obstetrics and gynaecology research 2012, 38, 435.
[6] M. Mehdizadeh, J. Yang, Macromol Biosci 2013, 13, 271.
[7] T. J. Guzzo, R. A. Pollock, A. Forney, P. Aggarwal, B. R. Matlaga, M. E. Allaf, Journal of endourology / Endourological Society 2009, 23, 279.
[8] A. Gugerell, K. Schossleitner, S. Wolbank, S. Nürnberger, H. Redl, H. Gulle, A. Goppelt, M. Bittner, W. Pasteiner, Journal of Biomedical Materials Research Part A 2012, 100A, 1239.
[9] (a) H. H. Versteeg, J. W. M. Heemskerk, M. Levi, P. H. Reitsma, Physiological Reviews 2013, 93, 327; (b) S. R. Coughlin, Nature 2000, 407, 258.
[10] H. P. Kohler, Blood 2013, 121, 1931.
[11] K. J. Clemetson, Thrombosis research 2012, 129, 220.
[12] S. R. Macfarlane, M. J. Seatter, T. Kanke, G. D. Hunter, R. Plevin, Pharmacological reviews 2001, 53, 245.
[13] H. Andersen, D. L. Greenberg, K. Fujikawa, W. Xu, D. W. Chung, E. W. Davie, Proceedings of the National Academy of Sciences of the United States of America 1999, 96, 11189.
[14] (a) S. R. Coughlin, Journal of thrombosis and haemostasis : JTH 2005, 3, 1800; (b) K. M. Austin, L. Covic, A. Kuliopulos, Blood 2013, 121, 431.
[15] R. R. Vassallo, Jr., T. Kieber-Emmons, K. Cichowski, L. F. Brass, The Journal of biological chemistry 1992, 267, 6081.
[16] (a) B. V. Slaughter, S. S. Khurshid, O. Z. Fisher, A. Khademhosseini, N. A. Peppas, Advanced materials 2009, 21, 3307; (b) N. A. Peppas, J. Z. Hilt, A. Khademhosseini, R. Langer, Adv. Mater. 2006, 18, 1345.
[17] R. H. Schmedlen, K. S. Masters, J. L. West, Biomaterials 2002, 23, 4325.
[18] C. E. Hoyle, C. N. Bowman, Angewandte Chemie 2010, 49, 1540; C. C. Lin, A. Raza, H. Shih, Biomaterials 2011, 32, 9685.
[18^{a}] Lin et al., J. Appl. Polym. Sci. 132 (2015), doi:10.1002/app.41563.
[18^{b}] Herner et al., Top. Curr. Cehm (Z) (2015), doi:10.1007/s41061-015-0002-2.
[19] B. D. Fairbanks, M. P. Schwartz, A. E. Halevi, C. R. Nuttelman, C. N. Bowman, K. S. Anseth, Adv. Mater. 2009, 21, 5005.
[20] (a) L. Hou, S. Ravenall, M. G. Macey, P. Harrlott, S. Kapas, G. L. Howells, Journal of Periodontal Research 1998, 33, 205; (b) N. Asokananthan, P. T. Graham, J. Fink, D. A. Knight, A. J. Bakker, A. S. McWilliam, P. J. Thompson, G. A. Stewart, J Immunol 2002, 168, 3577; (c) C. Keller, Y. Hellsten, A. Steensberg, B. K. Pedersen, Cytokine 2006, 36, 141.
[21] J. Zipperle, C. J. Schlimp, W. Holnthoner, A. M. Husa, S. Nurnberger, H. Redl, H. Schochl, Thrombosis and haemostasis 2013, 109, 869.
[22] H. Schöchl, C. Solomon, V. Laux, S. Heitmeier, S. Bahrami, H. Redl, Thrombosis research 2012, 130, e107.
[23] X.-H. Qin, A. Ovsianikov, J. Stampfl, R. Liska, BioNanoMaterials, Vol. 15, 2014, 49.
[24] A. M. Kloxin, C. J. Kloxin, C. N. Bowman, K. S. Anseth, Adv. Mater. 2010, 22, 3484.
[25] H. Shih, C. C. Lin, Biomacromolecules 2012, 13, 2003.
[26] N. Boknas, L. Faxalv, T. L. Lindahl, S. Ramstrom, Journal of thrombosis and haemostasis : JTH 2014, 12, 515.
[27] X.-H. Qin, J. Torgersen, R. Saf, S. Muhleder, N. Pucher, S. C. Ligon, W. Holnthoner, H. Redl, A. Ovsianikov, J. Stampfl, R. Liska, J Polym Sci Pol Chem 2013, 51, 4799.
[28] X.-H. Qin, P. Gruber, M. Markovic, B. Plochberger, E. Klotzsch, J. Stampfl, A. Ovsianikov, R. Liska, Polym. Chem. 2014, 5, 6523.
[29] Chan, L. C. & Cox, B. G.; The Journal of Organic Chemistry 72, 8863-8869, (2007).
[30] Mädler, S., Bich, C., Touboul, D. & Zenobi, R.; Journal of Mass Spectrometry 44, 694-706, (2009).
[31] Wong, S. Y. & Putnam, D.; Bioconjugate Chemistry 18, 970-982, (2007).
[32] Xiao, S.-J., Wieland, M. & Brunner, S.; Journal of Colloid and Interface Science 290, 172-183, (2005).
[33] Qin, X. H., Labuda, K., Chen, J., Hruschka, V., Khadem, A., Liska, R., Redl, H. & Slezak, P.; Adv Funct Mater 25, 6606-6617, (2015).
[34] Shu, J. Y., Panganiban, B. & Xu, T.; Annual Review of Physical Chemistry 64, 631-657, (2013).
[35] Hern, D. L. & Hubbell, J. A.; Journal of Biomedical Materials Research 39, 266-276, (1998).
[36] Vassallo, R. R., Kieber-Emmons, T., Cichowski, K. & Brass, L. F.; Journal of Biological Chemistry 267, 6081-6085, (1992).

## Claims

1. Hemostatic material, wherein a thrombin receptor activating agent is covalently coupled to a biocompatible matrix and wherein the thrombin receptor activating agent has a thrombin receptor activating activity after covalent coupling to the biocompatible matrix.

2. Hemostatic material according to claim 1, wherein the biocompatible matrix is a hemostatic matrix and selected from the group consisting of a biomaterial, preferably a protein, a biopolymer or a polysaccharide matrix, especially a collagen, gelatin, fibrin, starch or chitosan matrix; and a synthetic polymer, preferably a polyvinyl alcohol, polyethylene glycol, or poly(N-isopropylacrylamide).

3. Hemostatic material according to claim 1 or 2, wherein the thrombin receptor activating agent is a thrombin receptor activating peptide (TRAP), preferably TRAP8, TRAP7, TRAP6, TRAP1-41, SLIGKV (for PAR-2 (human)), TFRGAP (for PAR-3 (human)), GYPGQV (for PAR-4 (human)), or amidated forms thereof, as well as mixtures thereof.

4. Hemostatic material according to any one of claims 1 to 3, wherein the matrix is a sponge, a woven or non-woven fabric, a preformed shape, preferably as a cylinder or cone for tooth extraction, a particulate or granulate material or a sheet.

5. Hemostatic material according to any one of claims 1 to 4, wherein the matrix comprises polyvinyl alcohol.

6. Method for producing a hemostatic material according to any one of claims 1 to 5, wherein a thrombin receptor activating agent is covalently coupled to a biocompatible matrix; wherein the thrombin receptor activating agent is activated with peptide sequences with bioorthogonal groups and/or wherein the biocompatible matrix is activated with an ene group.

7. Method according to claim 6, wherein the thrombin receptor activating agent is a thrombin receptor activating peptide (TRAP), preferably TRAP8, TRAP7, TRAP6, TRAP1-41, SLIGKV (for PAR-2 (human)), TFRGAP (for PAR-3 (human)), GYPGQV (for PAR-4 (human)), or amidated forms thereof, as well as mixtures thereof.

8. Method according to claim 6 or 7, wherein the thrombin receptor activating agent is activated with peptide sequences with bioorthogonal groups, and wherein the bioorthogonal group is alkene, sulfhydryl, alkyne, azido, hydroazide, or hydrazine, especially a cysteine moiety with an -SH group.

9. Method according to any one of claim 6 to 8, wherein the biocompatible matrix is activated with an ene group, and wherein the ene group is norbornene, maleimide, allyl, vinyl ester, acrylate, vinyl carbonate, or methacrylate.

10. Method according to any one of claims 6 to 9, wherein the chemical coupling is performed by a photoreaction, preferably by photo-triggered click chemistry (photo-triggered biorthogonal reactions).

11. Method according to any one of claims 6 to 10, wherein the chemical coupling is performed by thiol-norbornene photo-click chemistry.

12. Hemostatic material according to any one of claims 1 to 5 for use in surgery and/or in the treatment of injuries and/or wounds.

13. Kit, preferably for use in surgery and/or in the treatment of injuries and/or wounds, comprising
- a hemostatic material according to any one of claims 1 to 5 and
- at least one administration device, preferably selected from the group buffer solution, especially a buffer solution containing Ca²⁺ ions, a syringe, a tube, a catheter, forceps, scissors, a sterilising pad or lotion.

14. Kit according to claim 13, wherein the buffer solution further comprises a component selected from the group anti-bacterial agent, coagulatively active agent, immunosuppressive agent, anti-inflammatory agent, anti-fibrinolytic agent, especially aprotinin or ECEA, growth factor, vitamin, cell, or mixtures thereof.

15. Kit according to claim 13, further comprising a container with a component selected from the group anti-bacterial agent, coagulatively active agent, immunosuppressive agent, anti-inflammatory agent, anti-fibrinolytic agent, especially aprotinin or ECEA, growth factor, vitamin, cell, or mixtures thereof.

## Patentansprüche

1. Hämostatisches Material, wobei ein Thrombinrezeptor-aktivierendes Mittel kovalent an eine biokompatible Matrix gekoppelt ist und wobei das Thrombinrezeptor-aktivierende Mittel nach kovalenter Kopplung an die biokompatible Matrix eine Thrombinrezeptor-aktivierende Aktivität hat.

2. Hämostatisches Material nach Anspruch 1, wobei die biokompatible Matrix eine hämostatische Matrix ist und aus der Gruppe bestehend aus einem Biomaterial, vorzugsweise einem Protein, einem Biopolymer oder einer Polysaccharidmatrix, insbesondere einer Kollagen-, Gelatine-, Fibrin-, Stärke- oder Chitosanmatrix; und einem synthetischen Polymer, vorzugsweise einem Polyvinylalkohol, Polyethylenglykol oder Poly(N-isopropylacrylamid), ausgewählt ist.

3. Hämostatisches Material nach Anspruch 1 oder 2, wobei das Thrombinrezeptor-aktivierende Mittel ein Thrombinrezeptor-aktivierendes Peptid (TRAP), vorzugsweise TRAP8, TRAP7, TRAP6, TRAP1-41, SLIGKV (für PAR-2 (human)), TFRGAP (für PAR-3 (human)), GYPGQV (für PAR-4 (human)) oder amidierte Formen davon sowie Gemische davon ist.

4. Hämostatisches Material nach einem der Ansprüche 1 bis 3, wobei die Matrix ein Schwamm, ein Gewebe oder Faservlies, eine vorgeformte Form, vorzugsweise als Zylinder oder Kegel zur Zahnextraktion, ein partikelförmiges oder granuliertes Material oder eine Folie ist.

5. Hämostatisches Material nach einem der Ansprüche 1 bis 4, wobei die Matrix Polyvinylalkohol aufweist.

6. Verfahren zur Herstellung eines hämostatischen Materials nach einem der Ansprüche 1 bis 5, wobei ein Thrombinrezeptor-aktivierendes Mittel kovalent an eine biokompatible Matrix gekoppelt ist; wobei das Thrombinrezeptor-aktivierende Mittel mit Peptidsequenzen mit bioorthogonalen Gruppen aktiviert wird und/oder wobei die biokompatible Matrix mit einer En-Gruppe aktiviert wird.

7. Verfahren nach Anspruch 6, wobei das Thrombinrezeptor-aktivierende Mittel ein Thrombinrezeptor-aktivierendes Peptid (TRAP), vorzugsweise TRAP8, TRAP7, TRAP6, TRAP1-41, SLIGKV (für PAR-2 (human)), TFRGAP (für PAR-3 (human)), GYPGQV (für PAR-4 (human)) oder amidierte Formen davon sowie Gemische davon ist.

8. Verfahren nach Anspruch 6 oder 7, wobei das Thrombinrezeptor-aktivierende Mittel mit Peptidsequenzen mit bioorthogonalen Gruppen aktiviert wird und wobei die bioorthogonale Gruppe Alken, Sulfhydryl, Alkin, Azid, Hydroazid oder Hydrazin, insbesondere ein Cysteinrest mit einer -SH-Gruppe, ist.

9. Verfahren nach einem der Ansprüche 6 bis 8, wobei die biokompatible Matrix mit einer En-Gruppe aktiviert wird und wobei die En-Gruppe Norbornen, Maleimid, Allyl, Vinylester, Acrylat, Vinylcarbonat oder Methacrylat ist.

10. Verfahren nach einem der Ansprüche 6 bis 9, wobei die chemische Kopplung durch eine Photoreaktion, vorzugsweise durch lichtinduzierte Klick-Chemie (lichtinduzierte bioorthogonale Reaktionen), durchgeführt wird.

11. Verfahren nach einem der Ansprüche 6 bis 10, wobei die chemische Kopplung durch Photo-Klick-Chemie mit Thiol-Norbornen durchgeführt wird.

12. Hämostatisches Material nach einem der Ansprüche 1 bis 5 zur Verwendung in der Chirurgie und/oder bei der Behandlung von Verletzungen und/oder Wunden.

13. Kit, vorzugsweise zur Verwendung in der Chirurgie und/oder bei der Behandlung von Verletzungen und/oder Wunden, aufweisend
- ein hämostatisches Material nach einem der Ansprüche 1 bis 5 und
- mindestens eine Verabreichungsvorrichtung, vorzugsweise ausgewählt aus der Gruppe bestehend aus Pufferlösung, insbesondere einer Pufferlösung, die Ca²⁺-Ionen enthält, einer Spritze, einem Schlauch, einem Katheter, einer Zange, einer Schere, einem Sterilisationstupfer oder einer Lotion.

14. Kit nach Anspruch 13, wobei die Pufferlösung ferner eine Komponente, ausgewählt aus der Gruppe bestehend aus antibakteriellem Mittel, gerinnungsförderndem Wirkstoff, immunsuppressivem Mittel, entzündungshemmendem Mittel, antifibrinolytischem Mittel, insbesondere Aprotinin oder ECEA, einem Wachstumsfaktor, Vitamin, einer Zelle oder Gemischen, davon aufweist

15. Kit nach Anspruch 13, ferner aufweisend einen Behälter mit einer Komponente, ausgewählt aus der Gruppe bestehend aus antibakteriellem Mittel, gerinnungsförderndem Wirkstoff, immunsuppressivem Mittel, entzündungshemmendem Mittel, antifibrinolytischem Mittel, insbesondere Aprotinin oder ECEA, einem Wachstumsfaktor, Vitamin, einer Zelle oder Gemischen davon.

## Revendications

1. Matériau hémostatique, dans lequel un agent d'activation de récepteur de thrombine est couplé par covalence à une matrice biocompatible et dans lequel l'agent d'activation de récepteur de thrombine présente une activité d'activation de récepteur de thrombine après couplage covalent à la matrice biocompatible.

2. Matériau hémostatique selon la revendication 1, dans lequel la matrice biocompatible est une matrice hémostatique et est sélectionnée à partir du groupe constitué par un biomatériau, de préférence, une protéine, un biopolymère ou une matrice polysaccharide, en particulier, un collagène, de la gélatine, de la fibrine, une matrice d'amidon ou de chitosane ; et un polymère synthétique, de préférence, un alcool polyvinylique, du polyéthylène glycol, ou du poly(N-isopropylacrylamide).

3. Matériau hémostatique selon la revendication 1 ou 2, dans lequel l'agent d'activation de récepteur de thrombine est un peptide d'activation de récepteur de thrombine (TRAP), de préférence, TRAP8, TRAP7, TRAP6, TRAP1-41, SLIGKV (pour récepteur activé par protéase PAR-2 (humain)), TFRGAP (pour PAR-3 (humain)), GYPGQV (pour PAR-4 (humain)), ou des formes amides de ceux-ci, de même que des mélanges de ceux-ci.

4. Matériau hémostatique selon l'une quelconque des revendications 1 à 3, dans lequel la matrice est une éponge, un tissu tissé ou non-tissé, une forme préformée, de préférence, telle qu'un cylindre ou un cône afin d'assurer l'extraction de dent, un matériau particulaire ou granulaire ou une feuille.

5. Matériau hémostatique selon l'une quelconque des revendications 1 à 4, dans lequel la matrice comprend de l'alcool polyvinylique.

6. Procédé de production d'un matériau hémostatique selon l'une quelconque des revendications 1 à 5, dans lequel un agent d'activation de récepteur de thrombine est couplé par covalence à une matrice biocompatible ; dans lequel l'agent d'activation de récepteur de thrombine est activé avec des séquences peptides comportant des groupes bioorthogonaux et/ dans lequel la matrice biocompatible est activée avec un groupe ène.

7. Procédé selon la revendication 6, dans lequel l'agent d'activation de récepteur de thrombine est un peptide d'activation de récepteur de thrombine (TRAP), de préférence, TRAP8, TRAP7, TRAP6, TRAP1-41, SLIGKV (pour PAR-2 (humain)), TFRGAP (pour PAR-3 (humain)), GYPGQV (pour PAR-4 (humain)), ou des formes amides de ceux-ci, de même que des mélanges de ceux-ci.

8. Procédé selon la revendication 6 ou 7, dans lequel l'agent d'activation de récepteur de thrombine est activé avec des séquences peptides comportant des groupes bioorthogonaux, et dans lequel le groupe bioorthogonal est un alcène, du sulfhydryle, de l'alcyne, de l'azido, de l'hydroazide, ou de l'hydrazine, en particulier, une fraction cystéine avec un groupe -SH.

9. Procédé selon l'une quelconque des revendications 6 à 8, dans lequel la matrice biocompatible est activée avec un groupe ène, et dans lequel le groupe ène est du norbornène, du maléimide, de l'allyle, de l'ester de vinyle, de l'acrylate, du carbonate de vinyle ou du méthacrylate.

10. Procédé selon l'une quelconque des revendications 6 à 9, dans lequel le couplage chimique est réalisé par photoréaction, de préférence, par chimie clic photo-induite (réactions biorthogonales photo-induites).

11. Procédé selon l'une quelconque des revendications 6 à 10, dans lequel le couplage chimique est réalisé par chimie photo-clic de thiol-norbornène.

12. Matériau hémostatique selon l'une quelconque des revendications 1 à 5 destiné à être utilisé en chirurgie et/ou pour le traitement de blessures et/ou de plaies.

13. Kit destiné, de préférence, à être utilisé en chirurgie et/ou pour le traitement de blessures et/ou de plaies, comprenant :
un matériau hémostatique selon l'une quelconque des revendications 1 à 5 et
au moins un dispositif d'administration sélectionné, de préférence, à partir du groupe comprenant une solution tampon, en particulier, une solution tampon contenant des ions Ca²⁺, une seringue, un tube, un cathéter, des forceps, des ciseaux, un tampon ou une lotion stérilisante.

14. Kit selon la revendication 13, dans lequel la solution tampon comprend, en outre, un composant sélectionné à partir du groupe constitué par un agent anti-bactérien, un agent coagulant, un agent immunosuppresseur, un agent anti-inflammatoire, un agent anti-fibrinolytique, en particulier, de l'aprotinine ou l'ECEA, un facteur de croissance, de la vitamine, une cellule ou des mélanges de ceux-ci.

15. Kit selon la revendication 13, comprenant, en outre, un conteneur avec un composant sélectionné à partir du groupe constitué par un agent anti-bactérien, un agent coagulant, un agent immunosuppresseur, un agent anti-inflammatoire, un agent anti-fibrinolytique, en particulier, de l'aprotinine ou l'ECEA, un facteur de croissance, de la vitamine, une cellule ou des mélanges de ceux-ci.
